# FASCICULE DE BREVET EUROPEEN

(11) **EP 2 249 847 B1**
(45) Date de publication et mention de la délivrance du brevet: **12.04.2017**
(21) Numéro de dépôt: 09720028.1
(22) Date de dépôt: 06.02.2009
(51) Int. Cl.: A61K 36/16, A61P 25/28

(54) **NOUVEAU PROCEDE POUR LA PREPARATION D'EXTRAITS DE GINKGO BILOBA**
NEUARTIGES VERFAHREN ZUR HERSTELLUNG VON EXTRAKTEN AUS GINKGO BILOBA
NOVEL METHOD FOR PREPARING EXTRACTS OF GINKGO BILOBA

(30) Priorité: 06.02.2008 FR 0800625
(43) Date de publication de la demande: 17.11.2010
(73) Titulaire: IPSEN PHARMA S.A.S., 92100 Boulogne-Billancourt (FR)
(72) Inventeur: TENG, Ben-Poon, F-91190 Gif-sur-Yvette (FR)
(74) Mandataire: Audonnet, Nathalie
(86) Numéro de dépôt international: PCT/FR2009/000137
(87) Numéro de publication internationale: WO 2009/112709

(56) Documents cités:
- WO-A-02/083158
- WO-A-2006/117168
- WO-A-2006/117170
- DATABASE WPI Week 19962 Thomson Scientific, London, GB; AN 1996-018388 XP002495405 & KR 94 002 796 B1 (SUN KYONG IND LTD) 2 avril 1994 (1994-04-02)
- DATABASE WPI Week 200739 Thomson Scientific, London, GB; AN 2007-404841 XP002495406 & CN 1 891 242 A (XUZHOU JIYUAN NATURAL HEALTHCARE PROD CO LTD) 10 janvier 2007 (2007-01-10)

## Description

La présente invention concerne un procédé pour la préparation d'un extrait de Ginkgo biloba substantiellement dépourvu de polluants organiques persistants. La présente invention concerne également un extrait de Ginkgo biloba substantiellement dépourvu de polluants organiques persistants, susceptible d'être obtenu par le procédé de la présente demande, ainsi que son utilisation en particulier dans le domaine thérapeutique.

En raison de leurs activités vasodilatatrice, anti-oxydante et neuroprotectrice démontrées, les extraits de Ginkgo biloba sont, depuis de nombreuses années, utilisés à des fins thérapeutiques et en particulier pour le traitement des désordres circulatoires périphériques ou cérébraux, de la démence ou les syndromes de la démence.

La présence de certains composants indésirables tels que les acides ginkgoliques est considérablement réduite au cours de certains procédés d'extraction. Cependant, en raison de la pollution environnementale actuelle, on ne peut exclure, quelle que soit l'origine des feuilles de Ginkgo biloba, la présence de polluant organique persistant (POP), aussi minime soit-elle. Car les POP sont des composés organiques qui résistent aux dégradations biologiques naturelles (persistance dans l'environnement) ; ils présentent également le risque d'entraîner des effets nuisibles pour la santé humaine et l'environnement et une capacité à s'accumuler dans les tissus des organismes vivants (bioaccumulation dans la chaîne alimentaire). De par leurs propriétés de persistance et de bioaccumulation, on peut retrouver des POP très loin de leurs lieux d'émission, transportés par les courants marins ou atmosphériques. En raison de leur propriété et afin de prévenir tout risque, il est souhaitable que les taux de POP soient les plus bas possibles.

Selon la présente invention, on entend par polluants organiques persistants (POP), les hydrocarbures aromatiques polycycliques (HAP ou PAH (polycyclic aromatic hydrocarbons)), les diphényles polychlorés (polychlorinated biphényls ou PCB), les dibenzodioxines polychlorées (polychlorinated dibenzodioxins ou PCDD) et les dibenzofuranes polychlorées (polychlorinated dibenzofurans ou PCDF). Parmi les PAH, on peut citer en particulier les composés aromatiques suivants : benzo(a) anthracène, chrysène, triphénylène[a], benzo(b)fluoranthène, benzo(k/j)fluoranthène, benzo(a)pyrène, indéno(1,2,3-cd)pyrène, dibenz(ah)anthracène, benzo(ghi)pérylène, dibenzo(al)-pyrène, dibenzo-(ai)-pyrène, dibenzo-(ah)-pyrène, dibenzo-(ae)-pyrène, cyclopenta(cd)pyrène et 5-méthylchrysène. Parmi les PCDD/F, on peut citer en particulier les composés suivants : 2,3,7,8-TétraCDF ("CDF" = chlorodibenzofuranne), 1,2,3,7,8-PentaCDF, 2,3,4,7,8-PentaCDF, 1,2,3,4,7,8-HexaCDF, 1,2,3,6,7,8-HexaCDF, 1,2,3,7,8,9-HexaCDF, 2,3,4,6,7,8-HexaCDF, 1,2,3,4,6,7,8-HeptaCDF, 1,2,3,4,7,8,9-HeptaCDF, OctaCDF,CDF et le 2,3,7,8-TétraCDD ("CDD" = chlorodibenzodioxine), 1,2,3,7,8-PentaCDD, 1,2,3,4,7,8-HexaCDD, 1,2,3,6,7,8-HexaCDD, 1,2,3,7,8,9-HexaCDD, 1,2,3,4,6,7,8-HeptaCDD et OctaCDD. Enfin parmi les PCB, on peut citer en particulier le PCB 77, PCB 81, PCB 105, PCB 114, PCB 118, PCB 123, PCB 126, PCB 156, PCB 157, PCB 167, PCB 169 et le PCB 189.

Les demandes KR 19890008341 et WO 02/083158 concernent des procédés d'extraction de feuilles de Ginkgo biloba pour l'obtention d'extraits enrichis mais ne font nullement mention d'une réduction de PAHs et/ou de POPs. Le brevet EP 431535 décrit un procédé d'extrait de Ginkgo biloba dont le but essentiel est d'obtenir un extrait avec un taux réduit d'acides ginkgoliques (taux inférieur à 10 ppm et de préférence à 1 ppm).

La demande WO 2006/117170 propose d'améliorer le procédé de préparation du brevet EP 431535 de sorte que l'extrait final ainsi obtenu présente un taux encore plus réduit d'hydrocarbures aromatiques polycycliques (HAP). Les demandes WO 2006/117168 et CN 20051080493 décrivent également des procédés d'obtention d'extrait de Ginkgo biloba avec un taux de PAH réduit mais les PAHs ne sont pas totalement éliminés.

La déposante a trouvé que les procédés tels que décrits dans le brevet EP 431535 et la demande WO 2006/117170 pouvaient encore être améliorés de sorte que l'extrait final ainsi obtenu soit substantiellement dépourvu des POPs tels que définis ci-dessus y compris les HAP.

La présente invention a donc pour objet un procédé d'obtention d'un extrait de feuilles de Ginkgo biloba substantiellement dépourvu de polluants organiques persistants, ledit procédé comprenant les étapes suivantes :
(a) les feuilles (de préférence vertes fraîches ou séchées) de Ginkgo biloba sont extraites à une température d'approximativement 40 à 100° C avec de l'acétone aqueux, un alcool aqueux de 1 à 3 atomes de carbone ou du méthanol anhydre ;
(b) la majeure partie du solvant organique est séparée de l'extrait à une teneur maximale de 10 % en poids, de sorte que de l'eau peut être ajoutée au cours des dernières étapes de distillation,
(c) la solution aqueuse concentrée restante est diluée avec de l'eau à une teneur en matières solides de 5 à 25 % en poids, laissée refroidir, tout en étant agitée, à une température en dessous de 25° C, laissée reposer jusqu'à ce qu'un précipité se forme et le précipité résultant, constitué des composants lipophiles qui ne se dissolvent pas bien dans l'eau, est séparé,
(d) du sulfate d'ammonium est ajouté à la solution aqueuse restante et la solution formée est extraite avec de la méthyléthylcétone ou un mélange de méthyléthylcétone et d'acétone,
(e) l'extrait obtenu est concentré à une teneur en matières solides de 50 à 70 % et le concentré obtenu est dilué avec de l'eau et de l'éthanol de manière à obtenir une solution qui contient 50 % en poids d'eau et 50 % en poids d'éthanol avec une teneur en matières solides de 10 % en poids,
(f) une solution aqueuse d'un sel de plomb est ajoutée à la solution ainsi obtenue jusqu'a ce qu'un changement de la couleur brune à la couleur ambre se produise, et le précipité formé est séparé, ou un polyamide est utilisé à la place d'un sel de plomb,
(g) la solution alcoolique aqueuse restante est extraite avec un solvant aliphatique ou cycloaliphatique avec un point d'ébullition de 60 à 100° C afin de séparer davantage les composés d'alkylphenol,
(h) la solution alcoolique aqueuse restante est concentrée sous pression réduite à une teneur en éthanol maximale d'approximativement 5 % et du sulfate d'ammonium est ajouté jusqu'à une teneur de 20 % en poids,
(i) la solution obtenue est extraite avec un mélange de méthyléthylcétone et d'éthanol dans un rapport de 8/2 à 5/5, de préférence de 6/4,
(j) la phase organique résultante est concentrée à une teneur en matières solides de 50 à 70 % en poids,
(k) au concentré ainsi obtenu, on ajoute de l'éthanol jusqu'à obtenir un contenu en éthanol d'au moins 80 %, puis on refroidit à une température maximale de 12° C pendant au moins deux heures et enfin les insolubles sont éliminés par filtration et on récupère le filtrat ;
(l) le filtrat est concentré et séché sous pression réduite à une température entre 60 à 80° C jusqu'à l'obtention d'un extrait sec avec une teneur en eau de moins de 5 %,
caractérisé en ce que le dit procédé comprend une étape supplémentaire qui consiste à faire passer une des phases organiques obtenues à l'une des étapes d, i ou k, sur un adsorbant qui est constitué de charbon actif présentant une surface spécifique supérieure ou égale à 1000 m²/g.

Selon la présente invention, l'utilisation d'adsorbant telle que définie par la présente demande permet la suppression de composés indésirables tels que les POP tout en maintenant le taux de composants actifs tels que flavones et de terpènes trilactones (ginkgolides et bilobalide).

La toxicité des POP varie suivant qu'il s'agit de PAH ou de PCDD/F et PCB. C'est ainsi que pour les composés de type PCDD/F et PCB, un système d"'équivalents toxiques" (TEQ) a été mis en place : ce coefficient de pondération indique le degré de toxicité par rapport au 2,3,7,8-TCDD (2,3,7,8-tétrachlorodibenzo-p-dioxine) (Van den Berg et al. (1998), Environmental Health Perspectives 106 (12), 775).

Par l'expression "extrait de Ginkgo biloba substantiellement dépourvu de polluants organiques persistants", on entend tout extrait de ginkgo biloba contenant un taux de polluants organiques persistants très réduit et de préférence tel que :
- le taux de chaque PAH est inférieur à 5 µg/kg, et de préférence à 1 µg/kg, et de préférence à 0,5 µg/kg et de manière très préférentielle inférieur à la limite de quantification (LOQ), et/ou
- le TEQ (PCDD/PCDF-PCB) est inférieur à 10 pg/g, de préférence à 1,5 pg/g, et de manière très préférentielle à 1,0 pg/g.

De préférence, le procédé ci-dessus est caractérisé en ce que
dans l'étape (b) le solvant organique est séparé à une teneur maximale de 5 % en poids,
dans l'étape (c) la solution aqueuse est diluée avec de l'eau à une teneur en matières solides de 15 à 20 % en poids et est refroidie a une température d'approximativement 10 à 12° C,
dans l'étape (d) le sulfate d'ammonium est ajouté à la solution pour donner une teneur de 30 % en poids et la solution formée est extraite avec le solvant dans un rapport de 9/1 à 4/6, de préférence de 6/4, et
dans l'étape (f) de l'acétate de plomb, de l'acétate basique de plomb ou du nitrate de plomb ou une solution aqueuse d'hydroxyde de plomb est utilisé.

De préférence également, le procédé ci-dessus est caractérisé en ce que, à l'étape (a),
les feuilles de Ginkgo biloba (de préférence sèches) sont extraites à une température comprise entre 50 et 65° C environ, avec comme solvant d'extraction soit l'acétone aqueuse avec un ratio en poids acétone/eau compris entre 50/50 et 65/35 et un ratio solides/solvant de 1/6 à 1/20 ; soit avec de l'éthanol aqueux,
et de manière préférentielle
le solvant d'extraction utilisé est l'acétone aqueuse avec un ratio en poids acétone/eau de 60/40 environ.

De préférence également, le procédé ci-dessus est caractérisé en ce que, à l'étape (b),
le solvant organique de l'extrait est éliminé par évaporation sous pression réduite jusqu'à un taux maximum de solvant restant inférieur à 5 % et jusqu'à obtenir un contenu solide de l'ordre de 15 à 30 %.

De préférence également, le procédé ci-dessus est caractérisé en ce que, à l'étape (c),
la solution aqueuse concentrée restante est diluée avec de l'eau jusqu'à obtenir un contenu solide de l'ordre de 10 à 20 % en poids et un taux maximum de solvant restant inférieur à 5 %, puis laissée se refroidir sous agitation à une température de l'ordre de 4-12° C pendant au moins une heure.

De préférence également, le procédé ci-dessus est caractérisé en ce que, à l'étape (d),
la solution aqueuse restante est extraite avec un mélange méthyléthylcétone/acétone avec un ratio en poids de 6/4 environ, en présence de 30 à 40 %, et de préférence 35 %, en poids par rapport au volume de la solution aqueuse, de sulfate d'ammonium, puis, après séparation de phases, on récupère la phase organique.

De préférence également, le procédé ci-dessus est caractérisé en ce que, à l'étape (f),
une solution aqueuse d'acétate basique de plomb (CH₃CO₂)₂Pb.2Pb(OH)₂ [CAS# 1335-32-6] à une concentration de l'ordre de 15 à 20 % est ajoutée à la solution ainsi obtenue à l'étape précédente (e), avec un ratio (quantité de Pb/contenu solide) compris entre 0,15 et 0,30, et de manière très préférentielle compris entre 0,18 et 0,25.

De préférence également, le procédé ci-dessus est caractérisé en ce que, à l'étape (g), la solution alcoolique aqueuse restante est extraite avec de l'heptane.

De préférence également, le procédé ci-dessus est caractérisé en ce qu'il comprend une étape supplémentaire qui consiste à faire passer sur un adsorbant la phase organique obtenue à l'étape d.

De préférence également, le procédé ci-dessus est caractérisé en ce qu'il comprend une étape supplémentaire qui consiste à faire passer sur un adsorbant la phase organique obtenue à l'étape i.

De préférence également, le procédé ci-dessus est caractérisé en ce qu'il comprend une étape supplémentaire qui consiste à faire passer sur un adsorbant la phase organique obtenue à l'étape k.

La présente invention a également pour objet un procédé d'obtention d'un extrait de feuilles de Ginkgo biloba substantiellement dépourvu de polluants organiques persistants, ledit procédé comprenant les étapes suivantes :
(aa) extraction de feuilles sèches de Ginkgo biloba à une température comprise entre 40 et 100° C environ, avec un solvant d'extraction choisi parmi : l'acétone aqueuse ou un alcool inférieur de 3 atomes de carbone aqueux ;
(ba) élimination du solvant organique de l'extrait jusqu'à un taux maximum de solvant restant inférieur à 10 % ;
(ca) la solution aqueuse concentrée restante est diluée avec de l'eau jusqu'à obtenir un contenu solide de l'ordre de 5 à 25 % en poids et un taux maximum de solvant restant inférieur à 5 %, puis laissée se refroidir sous agitation à une température inférieure à 25° C pendant au moins une heure et le précipité ainsi formé, consistant en des composés lipophiles, est éliminé ;
(da) la solution aqueuse restante est extraite avec un mélange méthyl éthyl cétone / acétone en présence de sulfate d'ammonium, puis, après séparation de phases, la phase organique est concentrée jusqu'à un contenu solide de 40 à 60 % en poids,
(ea) le concentré obtenu est dilué avec de l'éthanol aqueux et de l'eau de telle sorte que la solution finale contienne environ 50 % en poids d'eau et 50 % en poids d'éthanol et un contenu solide de l'ordre de 10-12 % ;
(fa) une solution aqueuse de sels de plomb est ajoutée à la solution ainsi obtenue à l'étape (e), avec un ratio (quantité de Pb/contenu solide) compris entre 0,15 et 0,3, et le précipité de plomb ainsi formé est retiré ;
(ga) la solution éthanolique aqueuse restante est extraite avec de l'heptane afin de retirer les composés de type alkylphénol et la solution aqueuse alcoolique restante est concentrée sous pression réduite jusqu'à un contenu d'éthanol inférieur à 5 % en poids ;
(ha) la solution obtenue est extraite avec un mélange méthyl éthyl cétone et éthanol, en présence de sulfate d'ammonium ;
(ia) à la phase organique obtenue à l'étape précédente est ajouté du sulfate d'ammonium sous agitation puis, après décantation, on récupère la phase organique qui est ensuite concentrée jusqu'à un contenu solide de l'ordre de 50-70 % en poids ;
(ja) au concentré ainsi obtenu, on ajoute de l'éthanol et de l'eau jusqu'à obtenir un contenu solide de 10 à 12 % , un contenu en éthanol d'environ 75 à 90 %, le reste étant de l'eau, puis on refroidit aux environs de 8-12° C pendant au moins une heure et enfin les insolubles sont éliminés par filtration et on récupère le filtrat ;
(ka) le filtrat ainsi obtenu est concentré sous pression réduite jusqu'à obtenir un contenu solide de 50 à 70 % en poids, et séché dans une étuve sous vide à une température qui ne dépasse pas 60° C ;
et une étape supplémentaire consistant à faire passer une des phases organiques obtenues à l'une des étapes précédentes da, ia ou ja, sur un adsorbant qui est constitué de charbon actif présentant une surface spécifique supérieure ou égale à 1000 m²/g.

De préférence, le procédé ci-dessus est caractérisé en ce que, à l'étape (aa),
l'extraction de feuilles sèches de Ginkgo biloba s'effectue à une température comprise entre 50-65° C environ, avec comme solvant d'extraction soit l'acétone aqueuse avec un ratio en poids acétone/eau compris entre 50/50 et 65/35 et un ratio (feuilles sèches/ solvant) compris entre 1/6 et 1/20, soit de l'éthanol aqueux,
et de manière très préférentielle
le solvant d'extraction utilisé est l'acétone aqueuse avec un ratio en poids acétone/eau de 60/40 environ.

De préférence également, le procédé ci-dessus est caractérisé en ce que, à l'étape (ba),
l'élimination du solvant organique de l'extrait s'effectue par évaporation sous pression réduite jusqu'à un taux maximum de solvant restant inférieur à 5 % et jusqu'à obtenir un contenu solide de l'ordre de 15 à 30 %.

De préférence également, le procédé ci-dessus est caractérisé en ce que, à l'étape (ca), la solution aqueuse concentrée restante est diluée avec de l'eau jusqu'à obtenir un contenu solide de l'ordre de 10 à 20 % en poids, puis laissée se refroidir sous agitation à une température de 4-12° C.

De préférence également, le procédé ci-dessus est caractérisé en ce que, à l'étape (da),
la solution aqueuse restante est extraite avec un mélange méthyle éthyle cétone / acétone avec un ratio en poids compris entre 5/5 et 7/3, de préférence de 6/4 environ, en présence de sulfate d'ammonium avec un taux de 30 à 40 %, et de préférence 35 %, en poids par rapport au volume de la solution aqueuse.

De préférence également, le procédé ci-dessus est caractérisé en ce que, à l'étape (fa),
le sel de plomb est choisi parmi l'acétate de plomb, l'hydroxyde de plomb et l'acétate basique de plomb.

De préférence également, le procédé ci-dessus est caractérisé en ce que, à l'étape (fa),
le ratio (Pb/contenu solide) est compris entre 0,18 et 0,25.

De préférence également, le procédé ci-dessus est caractérisé en ce que, à l'étape (fa),
une solution aqueuse d'acétate basique de plomb (CH₃CO₂)₂Pb.2Pb(OH)₂ [CAS# 1335-32-6] à une concentration de l'ordre de 15 à 20 %, est ajoutée à la solution obtenue à l'étape (ea), avec un ratio (quantité de Pb/contenu solide) compris entre 0,18 et 0,25.

De préférence également, le procédé ci-dessus est caractérisé en ce que, à l'étape (ha),
la solution obtenue est extraite avec un mélange méthyl éthyl cétone et éthanol avec un ratio de l'ordre de 6/4 en poids, en présence de 30 à 40 %, et de préférence 35 % en poids par rapport au volume de la solution aqueuse, de sulfate d'ammonium.

De préférence également, le procédé ci-dessus est caractérisé en ce que, à l'étape (ja), on ajoute de l'éthanol et de l'eau jusqu'à obtenir un contenu en éthanol d'environ 78 à 90 %, et de préférence 78 à 80%.

De préférence également, le procédé ci-dessus est caractérisé en ce qu'il comprend une étape supplémentaire qui consiste à faire passer sur un adsorbant la phase organique obtenue à l'étape da.

De préférence également, le procédé ci-dessus est caractérisé en ce qu'il comprend une étape supplémentaire qui consiste à faire passer sur un adsorbant la phase organique obtenue à l'étape ia.

De préférence également, le procédé ci-dessus est caractérisé en ce qu'il comprend une étape supplémentaire qui consiste à faire passer sur un adsorbant la phase organique obtenue à l'étape ja.

De préférence, les procédés ci-dessus sont caractérisés en ce que l'adsorbant comprend au moins l'une des caractéristiques ci-dessous :
- il ne comprend pas de groupe fonctionnel ;
- il se présente sous forme sphérique (ou de billes) ;
- il présente une surface spécifique supérieure ou égale à 1100 m²/g

De manière très préférentielle, les procédés ci-dessus sont caractérisés en ce que l'adsorbant comprend au moins deux des caractéristiques ci-dessous :
- il ne comprend pas de groupe fonctionnel ;
- il se présente sous forme sphérique (ou de billes) ;
- il présente une surface spécifique supérieure ou égale à 1100 m²/g

De manière très préférentielle également, les procédés ci-dessus sont caractérisés en ce que l'adsorbant est du charbon actif, sans groupe fonctionnel, sous forme sphérique (ou de billes), avec une surface spécifique minimale supérieure ou égale à 1100 m²/g.

De manière très préférentielle également, les procédés ci-dessus sont caractérisés en ce que l'adsorbant présente une taille de particules comprise entre 0,2 et 1 mm, et de préférence entre 0,3 et 0,9 mm.

De manière très préférentielle également, les procédés ci-dessus sont caractérisés en ce que l'adsorbant est du charbon actif, sans groupe fonctionnel, sous forme sphérique (ou de billes) de diamètre compris entre 0,4 et 0,8 mm, avec une surface spécifique minimale supérieure ou égale à 1200 m²/g avec des diamètres de pore d'environ 8 nm et des volumes de pore d'environ 0,15 cm³/g.

Selon la présente invention, l'adsorbant peut être sous forme sphérique (ou sous forme de bille) ; il présente donc une forme régulière. Pour l'homme de l'art, cette forme sphérique (ou "bead") se distingue des adsorbants de type granulaire ("granular"), avec une surface et/ou une forme irrégulière, ou bien des adsorbants se présentant sous forme cylindrique ("pellet" ou "rod") obtenus par extrusion.

L'adsorbant selon la présente invention est du charbon actif. Le charbon actif est naturel ou synthétique suivant la nature du produit de départ utilisé pour le préparer. Le charbon actif d'origine naturelle peut être produit à partir de toute matière organique naturelle riche en carbone telle que bois, écorces, coques de noix de coco, noyaux d'olives ou bien de résidus pétroliers. Le charbon actif d'origine synthétique peut être produit à partir de toute matière organique synthétique riche en carbone telle que le styrène, qui est soumise à pyrolyse sous conditions contrôlées, de préférence sous atmosphère inerte telle que sous azote, et à température élevée.

Du charbon actif sous forme sphérique peut être préparé à partir de billes préformées du produit de départ. Ainsi, du charbon actif synthétique sous forme sphérique peut être préparé à partir de billes préformées du produit de départ.

De préférence, le charbon actif utilisé selon la présente invention est un charbon actif synthétique.

La présente invention a également pour objet un extrait de Ginkgo biloba substantiellement dépourvu de polluants organiques persistants et susceptible d'être obtenu selon un des procédés tels que décrits ci-dessus.

De préférence, l'extrait tel que défini ci-dessus, substantiellement dépourvu de polluants organiques persistants, comprend
- 20 à 30 % en poids de flavones glycosides,
- 2,5 à 4,5 % en poids total de Ginkgolides A, B, C et J (au total),
- 2,0 à 4,0 % en poids de bilobalide,
- moins de 10 ppm d'alkylphénols et moins de 10 % en poids de proanthocyanidines ;
et de manière très préférentielle
- 22 à 26 % en poids de flavones glycosides,
- 2,5 à 4,5 % en poids total de Ginkgolides A, B, C et J,
- 2,0 à 4,0 % en poids de bilobalide,
- moins de 1 ppm d'alkylphénols et moins de 10 % en poids de proanthocyanidines.

De préférence également, l'extrait tel que défini ci-dessus, substantiellement dépourvu de polluants organiques persistants, comprend
- 20 à 30 % en poids de flavones glycosides,
- 2,5 à 4,5 % en poids total de Ginkgolides A, B et C,
- 2,0 à 4,0 % en poids de bilobalide,
- moins de 10 ppm d'alkylphénols et moins de 10 % en poids de proanthocyanidines.

De manière très préférentielle, les extraits tels que définis ci-dessus, comprennent un taux de polluants organiques persistants tel que
- le taux de chaque PAH est inférieur à 5 µg/kg, de préférence à 1 µg/kg et de manière très préférentielle à 0,5 µg/kg, et/ou
- le TEQ (PCDD/PCDF-PCB) est inférieur à 10 pg/g, de préférence à 1,5 pg/g;
et, de manière très préférentielle,
- le taux de chaque PAH est inférieur à la limite de quantification (LOQ), et/ou
- le TEQ (PCDD/PCDF-PCB) est inférieur à 1,0 pg/g.

Un extrait de Ginkgo biloba selon la présente invention peut être utilisé comme principe actif de composition thérapeutique ou bien comme additif alimentaire. Les compositions à base d'un tel extrait peuvent être sous forme de poudres, granules, comprimés ou gélules.

La présente invention a également pour objet l'utilisation d'un extrait tel que défini ci-dessus pour la préparation d'un médicament pour le traitement des désordres circulatoires périphériques ou cérébraux, de la démence ou les syndromes de la démence.

La présente invention a également pour objet une composition thérapeutique contenant, à titre de principe actif, un extrait tel que défini ci-dessus, en association avec des excipients thérapeutiquement acceptables.

En l'absence d'autre précision, les ratios et les pourcentages mentionnés dans la présente demande sont des ratios poids/poids et des pourcentages en poids. Bien entendu, les températures de mise en oeuvre des différentes étapes du procédé sont inférieures aux températures d'ébullition des solvants organiques utilisés, et de préférence au moins 5° C en dessous de la température d'ébullition.

### Partie expérimentale

### Exemple 1 : préparation d'un extrait de Ginkgo biloba

2 kg de feuilles sèches de Ginkgo biloba sont extraits deux fois, à une température d'environ 60° C, avec de l'acétone / eau (60/40 en poids) et en utilisant à chaque fois 10 fois plus de solvant (poids/poids).

Le solvant organique est éliminé par évaporation jusqu'à obtenir un contenu solide d'environ 35 %.

La solution aqueuse concentrée restante est diluée de moitié avec de l'eau jusqu'à obtenir un contenu solide de l'ordre de 17 % en poids, puis laissée se refroidir sous agitation à une température d'environ 10-12° C pendant au moins une heure et le précipité ainsi formé est éliminé.

La solution aqueuse restante est extraite avec un mélange méthyle éthyle cétone / acétone (6/4 en poids) en présence de 35 % (en poids) de sulfate d'ammonium, puis, après séparation de phases, de nouveau extraite avec un mélange méthyle éthyle cétone/acétone (6/4 en poids) avant d'être concentrée jusqu'à un contenu solide de 50 à 60 % en poids.

Le concentré obtenu est dilué avec de l'eau et de l'éthanol jusqu'à un ratio en poids (contenu solide/éthanol/eau) de (10/45/45). A la solution ainsi obtenue, on ajoute alors une solution aqueuse d'acétate basique de plomb (CH₃CO₂)₂Pb.2Pb(OH)₂ [CAS# 1335-32-6] à 16% avec un ratio en volume (solution de plomb/solution éthanolique) de (16/100) ; le précipité de plomb ainsi formé est retiré par centrifugation.

Le surnageant éthanolique aqueux récupéré est extrait trois fois avec de l'heptane avec à chaque fois 1/3 du volume total, afin de retirer les composés de type alkylphénol et la solution aqueuse alcoolique restante est concentrée sous pression réduite jusqu'à un contenu d'éthanol inférieur à 5 % en poids.

La solution aqueuse restante est extraite avec un mélange méthyle éthyle cétone/éthanol (6/4 en poids) en présence de 35 % (en poids) de sulfate d'ammonium, puis, après séparation de phases, de nouveau extraite avec un mélange méthyle éthyle cétone/acétone (6/4 en poids) ; les phases organiques sont rassemblées et passées sur colonne de charbon actif Lewatit AF5 (10 g).

A la phase organique obtenue à l'étape précédente est ajouté de nouveau 20 % (en poids) de sulfate d'ammonium sous agitation puis, après décantation, la phase organique est récupérée puis concentrée jusqu'à un contenu solide de l'ordre de 50-70 % en poids.

On ajoute ensuite au concentré ainsi obtenu de l'éthanol à plus de 90 % et de l'eau jusqu'à un ratio en poids (contenu solide/éthanol/eau) de (12/78/10), puis on refroidit aux environs de 8-12° C pendant au moins trois heures. Les insolubles sont ensuite filtrés et le filtrat est concentré sous pression réduite puis séché dans une étuve sous vide jusqu'à obtenir un contenu solide de plus de 98 % en poids.

### Exemple 2 : réduction du taux de POPs par passage sur adsorbant

Différentes solutions organiques d'un extrait de Ginkgo biloba volontairement contaminées par des POPs sont préparées puis passées sur adsorbant.

### i) Préparation de solutions d'extrait de Ginkgo biloba

Des solutions d'extrait standardisé de Ginkgo biloba (EGb761®) sont préparées dans trois solvants différents et volontairement contaminées par des POPs.
Solution N° 1 : 120 g d'extrait sec dans 1440 ml d'un mélange méthyl éthyl cétone/éthanol (6/4 en poids) et contenant 22 % en poids d'eau ; la solution ainsi obtenue est divisée en 2 volumes égaux.
Solution N° 2 : 60 g d'extrait sec dans 720 ml d'un mélange méthyl éthyl cétone/acétone (6/4 en poids) et contenant 20 % en poids d'eau ;
Solution N° 3 : 60 g d'extrait sec dans 720 ml d'éthanol aqueux (88 % en poids).

Quelle que soit la solution, le niveau de contamination souhaité ainsi que la limite de quantification sont les suivants :

| Contaminants ajoutés* | Niveau de contamination souhaité | Limite de quantification |
|---|---|---|
| PAH | 20 µg/kg | < 0,5 µg/kg |
| PCDD/PCDF | 200 ng/kg | < 0,1 à 0,4 ng/kg |
| PCB | 200 ng/kg | < 0,5 à 30 ng/kg |

| | | |
|---|---|---|
| * fournisseurs Supelco et Well-Labs | | |

Pour les PAH, les contaminants utilisés sont les suivants : benzo(a) anthracène, chrysène, triphénylène[a], benzo(b)fluoranthène, benzo(k/j)fluoranthène, benzo(a)pyrène, indéno(1,2,3-cd)pyrène, dibenz(ah)anthracéne, benzo(ghi)pérylène, dibenzo(al)-pyrène, dibenzo-(ai)-pyrène, dibenzo-(ah)-pyrène, dibenzo-(ae)-pyrène, cyclopenta(cd)pyrène et 5-méthylchrysène. Les résultats sont rassemblés dans le tableau 1.

Pour les PCDD/F, les contaminants utilisés sont les suivants : 2,3,7,8-TétraCDF, 1,2,3,7,8-PentaCDF, 2,3,4,7,8-PentaCDF, 1,2,3,4,7,8-HexaCDF, 1,2,3,6,7,8-HexaCDF, 1,2,3,7,8,9-HexaCDF, 2,3,4,6,7,8-HexaCDF, 1,2,3,4,6,7,8-HeptaCDF, 1,2,3,4,7,8,9-HeptaCDF, OctaCDF, 2,3,7,8-TétraCDD, 1,2,3,7,8-PentaCDD, 1,2,3,4,7,8-HexaCDD, 1,2,3,6,7,8-HexaCDD, 1,2,3,7,8,9-HexaCDD, 1,2,3,4,6,7,8-HeptaCDD et OctaCDD. Les résultats sont rassemblés dans le tableau 2.

Pour les PCB, les contaminants utilisés sont les suivants : PCB 77, PCB 81, PCB 105, PCB 114, PCB 118, PCB 123, PCB 126, PCB 156, PCB 157, PCB 167, PCB 169 et PCB 189. Les résultats sont rassemblés dans le tableau 3.

### ii) Passage des solutions contaminées sur un adsorbant

Les solutions contaminées sont passées sur l'adsorbant Lewatit AF5® (charbon actif synthétique sans groupe fonctionnel, sous forme sphérique, d'une surface spécifique minimum de 1200 m²/g, et dont au moins 90 % des particules présentent une taille entre 0,4 et 0,8 mm, et nommé ci-après "AF5") : les solutions sont passées 3 fois sur une colonne (diamètre interne 1 cm, hauteur de lit 20 cm) contenant 10 g de AF5.

Le ratio en poids entre l'extrait sec et l'adsorbant utilisé est environ de 6 sur 1.

### iii) Les résultats

Les résultats obtenus sont rassemblés dans les tableaux 1, 2 et 3 ci-dessous (les valeurs sont arrondies au maximum à 3 chiffres après la virgule).

Les légendes utilisées sont les suivantes :
<0,5 : signifie que la limite indiquée de quantification (LOQ) est de 0,5 et que la concentration trouvée est inférieure à cette limite.

ND : signifie Non Déterminé car aucun des congénères correspondants n'est au dessus du LOQ.

TEQ excl.LOQ[a] = valeur du TEQ calculée en incluant seulement les congénères quantifiés.

TEQ incl.LOQ[b] = valeur du TEQ calculée en incluant les congénères quantifiés en prenant les valeurs de leur LOQ.

Les résultats montrent que le passage sur l'adsorbant réduit le taux de POP avec un taux inférieur à la limite de quantification.

### Exemple 3 : exemple de référence

Les exemples 3a et 3b ci-dessous illustrent l'utilisation d'autre adsorbant et/ou solvants que ceux utilisés dans les exemples précédents. Le ratio en poids entre l'extrait sec et l'adsorbant utilisé est environ de 6 sur 1.

### Exemple 3a : utilisation d'une résine synthétique

Une résine synthétique est utilisée avec la solution N° 2 telle que définie à l'exemple 2 ci-dessus.

Les caractéristiques de cette résine sont les suivantes : résine Lewatit® VP OC 1163, sans groupement fonctionnel, à base de polystyrènes réticulés, sous forme sphérique, d'une surface spécifique supérieure à 1300 m²/g; avec une taille de particules comprises entre 0,45 et 0,55 mm.

Le protocole expérimental (contamination, passage de la solution sur l'adsorbant) est identique à celui décrit à l'exemple 2.

Les résultats obtenus sont rassemblés dans le tableau 4 ci-dessous (les valeurs sont arrondies au maximum à 3 chiffres après la virgule).

Ces résultats montrent qu'après le passage sur la résine, environ 25 à 70 % des PAHs ajoutés restent dans l'extrait alors que le passage sur l'adsorbant Lewatit AF5® (cf. exemple 1) réduit le taux de POP à un taux inférieur à la limite de quantification.

### Exemple 3b :

Le charbon actif utilisé est identique à celui de l'exemple 2 (Lewatit AF5®) avec une solution N°**4**.

Solution N° **4** : 120 g d'extrait sec dans 1440 ml de n-butanol et contenant 20 % en poids d'eau ; la solution ainsi obtenue est filtrée, les POPs sont ajoutés au filtrat et le volume total est divisé en 2 volumes égaux, dont un est utilisé comme contrôle et l'autre est passé sur l'adsorbant Lewatit AF5®.

Le protocole expérimental (contamination, passage de la solution sur l'adsorbant) est identique à celui décrit à l'exemple 2.

Les résultats obtenus sont rassemblés dans le tableau 5 ci-dessous (les valeurs sont arrondies au maximum à 3 chiffres après la virgule).

Avec un tel adsorbant, les résultats montrent qu'après le passage sur celui, environ 3 à 9 % de PAHs ajoutés, 1 à 12 % de PCDD/PCDF ajoutés et 27 à 46 % de PCBs ajoutés restent dans l'extrait alors que le passage sur l'adsorbant Lewatit AF5® (cf. exemple 2) réduit le taux de POP avec un taux inférieur à la limite de quantification.

Les résultats ainsi obtenus montrent qu'après le passage sur ce charbon actif mais avec du n-butanol comme solvant, environ 10 à 30 % de PCDD/PCDF ajoutés et 20 à 30 % de PCBs ajoutés restent dans l'extrait alors que le passage sur ce même adsorbant (cf. exemple 2) mais avec des solvants tels qu'utilisé à l'exemple 2, réduit le taux de POP avec un taux inférieur à la limite de quantification.

Les solvants utilisés à l'exemple 2, à savoir méthyl-éthyl-cétone, éthanol, acétone, eau ou des mélanges de ces derniers, présentent un indice de polarité (ou PI (Polarity Index), cf. Modem Methods of Pharmaceutical Analysis par Roger E. Schimer, Volume II, page 305 avec pour référence la valeur de 9 pour l'indice de polarité de l'eau) compris entre 5 et 6. Le n-butanol est moins polaire avec un indice de polarité de 4,8, c'est-à-dire en dehors de la fourchette 5-6.

Dans son ouvrage, Schimer précise que bien que la polarité d'un solvant pour un mélange de solvant ne dépende pas de façon linéaire de la composition, l'approximation linéaire est suffisamment exacte pour permettre son utilisation pour estimer l'index de polarité de mélanges. Ainsi l'index de polarité d'un mélange peut être calculé comme la somme des produits des fractions en volume et des index de polarité de chaque solvant qui constitue le mélange.

Pour un mélange de solvants A et B dont les fractions en volume par rapport au mélange sont V_{A} et V_{B} et les index de polarité sont respectivement I_{A} et I_{B} pour les solvants A et B purs, l'index de polarité du mélange est donc V_{A}I_{A} + V_{B}I_{B}.

**Tableau 1 : mesure des PAH**

| | | **Solution N°1** | **Solution N°1** | **Solution N°2** | **Solution N°3** |
|---|---|---|---|---|---|
| **Adsorbant utilisé** | | **aucun** | **AF 5** | **AF 5** | **AF5** |
| | | **µg/Kg** | **µg/Kg** | **µg/Kg** | **µg/Kg** |
| 1 | Benzo(a) anthracene | 24 | < 0,5 | < 0,5 | < 0,5 |
| 2 | Chrysene/Triphenylene[a] | 24 | < 0,5 | < 0,5 | < 0,5 |
| 3 | Benzo(b)fluoranthene | 29 | < 0,5 | < 0,5 | < 0,5 |
| 4 | Benzo(k/j)fluoranthene | 34 | < 0,5 | < 0,5 | < 0,5 |
| 5 | Benzo(a)pyrene | 26 | < 0,5 | < 0,5 | < 0,5 |
| 6 | Indeno(1,2,3-cd)pyrene | 25 | < 0,5 | < 0,5 | < 0,5 |
| 7 | Dihenz(ah)anthracene | 25 | < 0,5 | < 0,5 | < 0,5 |
| 8 | Benzo(ghi)perylene | 26 | < 0,5 | < 0,5 | < 0,5 |
| 9 | Dibenzo(al)-pyrene | 13 | <1 | <1 | <1 |
| 10 | Dibenzo-(ai)-pyrene | 9,7 | <1 | <1 | <1 |
| 11 | Dibenzo-(ah)-pyrene | 7,4 | <1 | <1 | <1 |
| 12 | Dibenzo-(ae)-pyrene | 8,1 | <1 | <1 | <1 |
| 13 | Cyclopenta(cd)pyrene | 13 | <1 | <1 | <1 |
| 14 | 5-Methylchrysen | 14 | <1 | <1 | <1 |

**Tableau 2 : mesure des PCDD/F**

| | **Solution N° 1** | **Solution N° 1** | **Solution N° 2** | **Solution N° 3** |
|---|---|---|---|---|
| **Adsorbant utilisé :** | **aucun** | **AF 5** | **AF 5** | **AF 5** |
| **PCDD/F** | **pg/g** | **pg/g** | **pg/g** | **pg/g** |
| 2,3,7,8-TetraCDF | 26 | <0,09 | <0,09 | <0,11 |
| 1,2,3,7,8-PentaCDF | 1124 | <0,12 | <0,13 | <0,14 |
| 2,3,4,7,8-PentaCDF | 114 | <0,12 | <0,13 | <0,14 |
| 1,2,3,4,7,8-HexaCDF | 101 | <0,11 | <0,12 | <0,13 |
| 1,2,3,6,7,8-HexaCDF | 80,2 | <0,11 | <0,12 | <0,13 |
| 1,2,3,7,8,9-HexaCDF | 122 | <0,11 | <0,12 | <0,13 |
| 2,3,4,6,7,8-HexaCDF | 99,8 | <0,11 | <0,12 | <0,13 |
| 1,2,3,4,6,7,8-HeptaCDF | 81,7 | <0,11 | <0,11 | <0,13 |
| 1,2,3,4,7,8,9-HeptaCDF | 102 | <0,11 | <0,11 | <0,13 |
| OctaCDF | 186 | <0,42 | <0,44 | <0,50 |
| **2378-TetraCDD** | 122 | <0,05 | <0,05 | <0,06 |
| 1,2,3,7,8-PentaCDD | 113 | <0,07 | <0,07 | <0,08 |
| 1,2,3,4,7,8-HexaCDD | 98,3 | <0,13 | <0,14 | <0,16 |
| 1,2,3,6,7,8-HexaCDD | 91,8 | <0,13 | <0,14 | <0,16 |
| 1,2,3,7,8,9-HexaCDD | 133 | <0,13 | <0,14 | <0,16 |
| 1,2,3,4,6,7,8-HeptaCDD | 87,9 | <0,09 | <0,09 | <0,11 |
| OctaCDD | 160 | 0,31 | 0,25 | 0,31 |
| | | | | |
| | | | | |
| OMS(2005)-PCDD/F-TEQ excl.LOQ[a] | 350 | 0,00009 | 0,00008 | 0,00009 |
| OMS(2005)-PCDD/F-TEQ incl.LOQ[b] | 350 | 0,255 | 0,268 | 0,305 |

**Tableau 3 : mesure des PCB**

| | **Solution N° 1** | **Solution N° 1** | **Solution N° 2** | **Solution N° 3** |
|---|---|---|---|---|
| **Adsorbant utilisé :** | **aucun** | **AF5** | **AF 5** | **AF5** |
| **OMS-PCB** | **pg/g** | **pg/g** | **pg/g** | **pg/g** |
| TetraCB (# 77) | 118 | <2,80 | <2,95 | <3,35 |
| TetraCB (# 81) | 111 | <0,56 | <0,59 | <0,67 |
| PentaCB (# 105) | 112 | <11,2 | <11,8 | <13,4 |
| PentaCB (# 114) | 95,8 | <1,2 | 1,35 | <1,34 |
| PentaCB (# 118) | 128 | <30,8 | <32,4 | <36,8 |
| PentaCB (# 123) | 104 | <2,80 | <2,95 | <3,35 |
| PentaCB (# 126) | 108 | <0,42 | <0,44 | <0,50 |
| HexaCB (# 156) | 91,4 | <5,60 | <5,90 | <6,70 |
| HexaCB (# 157) | 91,7 | <2,8 | <2,95 | <3,35 |
| HexaCB (# 167) | 88,5 | <2,8 | <2,95 | <3,35 |
| HexaCB (# 169) | 87,1 | <0,70 | <0,74 | <0,84 |
| HeptaCB (# 189) | 77,3 | <2,8 | <2,95 | <3,35 |
| PCB-TEQ : | | | | |
| OMS (2005)-PCB-TEQ excl.LOQ[a] | 13,5 | ND | 0,00004 | ND |
| OMS (2005)-PCB-TEQ incl.LOQ[b] | 13,5 | 0,065 | 0,069 | 0,078 |
| PCDD/F-PCB-TEQ: | | | | |
| OMS (2005)-PCDD/F-PCB-TEQ excl.LOQ[a] | 363,5 | 0,00009 | 0,00012 | 0,00009 |
| OMS(2005)-PCDD/F-PCB-TEQ incl.LOQ[b] | 363,5 | 0,320 | 0,337 | 0,383 |

**Tableau 4 (exemple 3a) :**

| | **Sans passage sur l'adsorbant** | **Après passage sur adsorbant** | **% restant dans l'extrait** |
|---|---|---|---|
| **PAHs** | ***µg*/*kg*** | ***µg*/*kg*** | |
| Fluorene | < 0,5 | 7,2 | |
| Phenanthrene | 9 | 2,3 | 25,6 |
| Anthracene | 4,9 | 3,4 | 69,4 |
| Fluoranthene | 3,4 | 1,4 | 41,2 |
| Pyrene | 13 | 8,9 | 68,5 |
| Benz(a)anthracene | 12 | 8,0 | 66,7 |
| Chrysene/Triphenylene[a] | 13 | 7,9 | 60,8 |
| Benzo(b)fluoranthene | 13 | 6,3 | 48,5 |
| Benzo(k)fluoranthene | 12 | 6,5 | 54,2 |
| **Benzo(a)pyrene** | 11 | 5,9 | 53,6 |
| Dibenz(ah)anthracene | 12 | 5,9 | 49,2 |
| Benzo(ghi)perylene | 11 | 4,8 | 43,6 |
| Indeno(1,2,3-cd)pxrene | 12 | 5,1 | 42,5 |
| **Total PAH excl.LOQ[b]** | **126,3** | **73,6** | |
| **Total PAH incl.LOQ[c]** | **126,8** | **73,6** | |

**Tableau 5 (exemple 3d) :**

| | **Sans passage sur l'adsorbant** | **Après passage sur adsorbant** | % restant dans l'extrait |
|---|---|---|---|
| **PCDD/F** | **ng/Kg** | **ng/Kg** | |
| 2378-TetraCDF | 3,69 | 0,42 | 11 |
| 12378-PentaCDF | 12,7 | 1,51 | 12 |
| 23478-PentaCDF | 13,6 | 2,58 | 19 |
| 123478-HexaCDF | 11,7 | 3,14 | 27 |
| 123678-HexaCDF | 9,58 | 2,41 | 25 |
| 123789-HexaCDF | 14,1 | 1,47 | 10 |
| 234678-HexaCDF | 12,1 | 3,3 | 27 |
| 1234678-HeptaCDF | 8,64 | 2,66 | 31 |
| 1234789-HeptaCDF | 12,2 | 2,47 | 20 |
| OctaCDF | 21,5 | 5,66 | 26 |
| **2378-TetraCDD** | 16,5 | 3,06 | 19 |
| 12378-PentaCDD | 12,8 | 2,07 | 16 |
| 123478-HexaCDD | 12,2 | 3,05 | 25 |
| 123678-HexaCDD | 12,1 | 2,57 | 21 |
| 123789-HexaCDD | 11,3 | 1,83 | 16 |
| 1234678-HeptaCDD | 10,9 | 2,71 | 25 |
| OctaCDD | 18,3 | 5,41 | 30 |

**Tableau 5 (exemple 3d) (suite) :**

| **WHO-PCB** | **ng/Kg** | **ng/Kg** | |
|---|---|---|---|
| TetraCB (# 77) | 23,1 | 5,04 | 22 |
| TetraCB (# 81) | 18,1 | 4,71 | 26 |
| PentaCB (# 105) | 16,8 | <11,6 | |
| PentaCB (# 114) | 15,7 | 5,42 | 35 |
| PentaCB (# 118) | <39,1 | <41,3 | |
| PentaCB (# 123) | 17,8 | 5,66 | 32 |
| PentaCB (# 126) | 17,6 | 3,68 | 21 |
| HexaCB (# 156) | 14,3 | <7,68 | |
| HexaCB (# 157) | 13,5 | 5,24 | 39 |
| HexaCB (# 167) | 15,4 | 5,45 | 35 |
| HexaCB (# 169) | 13,9 | 3,94 | 28 |
| HeptaCB (# 189) | 11,8 | 4,28 | 36 |

## Revendications

1. Procédé d'obtention d'un extrait de feuilles de Ginkgo biloba substantiellement dépourvu de polluants organiques persistants, ledit procédé comprenant les étapes suivantes :
(a) les feuilles de Ginkgo biloba sont extraites à une température d'approximativement 40 à 100° C avec de l'acétone aqueux, un alcool aqueux de 1 à 3 atomes de carbone ou du méthanol anhydre ;
(b) la majeure partie du solvant organique est séparée de l'extrait à une teneur maximale de 10 % en poids, de sorte que de l'eau peut être ajoutée au cours des dernières étapes de distillation,
(c) la solution aqueuse concentrée restante est diluée avec de l'eau à une teneur en matières solides de 5 à 25 % en poids, laissée refroidir, tout en étant agitée, à une température en dessous de 25° C, laissée reposer jusqu'à ce qu'un précipité se forme et le précipité résultant, constitué des composants lipophiles qui ne se dissolvent pas bien dans l'eau, est séparé,
(d) du sulfate d'ammonium est ajouté à la solution aqueuse restante et la solution formée est extraite avec de la méthyléthylcétone ou un mélange de méthyléthylcétone et d'acétone,
(e) l'extrait obtenu est concentré à une teneur en matières solides de 50 à 70 % et le concentré obtenu est dilué avec de l'eau et de l'éthanol de manière à obtenir une solution qui contient 50 % en poids d'eau et 50 % en poids d'éthanol avec une teneur en matières solides de 10 % en poids,
(f) une solution aqueuse d'un sel de plomb est ajoutée à la solution ainsi obtenue jusqu'à ce qu'un changement de la couleur brune à la couleur ambre se produise, et le précipité formé est séparé, ou un polyamide est utilisé à la place d'un sel de plomb,
(g) la solution alcoolique aqueuse restante est extraite avec un solvant aliphatique ou cycloaliphatique avec un point d'ébullition de 60 à 100° C afin de séparer davantage les composés d'alkylphenol,
(h) la solution alcoolique aqueuse restante est concentrée sous pression réduite à une teneur en éthanol maximale d'approximativement 5 % et du sulfate d'ammonium est ajouté jusqu'à une teneur de 20 % en poids,
(i) la solution obtenue est extraite avec un mélange de méthyléthylcétone et d'éthanol dans un rapport de 8/2 à 5/5, de préférence de 6/4,
(j) la phase organique résultante est concentrée à une teneur en matières solides de 50 à 70 % en poids,
(k) au concentré ainsi obtenu, on ajoute de l'éthanol jusqu'à obtenir un contenu en éthanol d'au moins 80 %, puis on refroidit à une température maximale de 12° C pendant au moins deux heures et enfin les insolubles sont éliminés par filtration et on récupère le filtrat ;
(l) le filtrat est concentré et séché sous pression réduite à une température entre 60 à 80° C jusqu'à l'obtention d'un extrait sec avec une teneur en eau de moins de 5 %,
**caractérisé en ce que** le dit procédé comprend une étape supplémentaire qui consiste à faire passer une des phases organiques obtenues à l'une des étapes d, i ou k, sur un adsorbant qui est constitué de charbon actif synthétique, présentant une surface spécifique supérieure ou égale à 1000 m²/g, avec un solvant ou mélange de solvants dont la polarité est comprise entre 5 et 6.

2. Procédé suivant la revendication 1, **caractérisé en ce que**
dans l'étape (b) le solvant organique est séparé à une teneur maximale de 5 % en poids,
dans l'étape (c) la solution aqueuse est diluée avec de l'eau à une teneur en matières solides de 15 à 20 % en poids et est refroidie a une température d'approximativement 10 à 12° C,
dans l'étape (d) le sulfate d'ammonium est ajouté à la solution pour donner une teneur de 30 % en poids et la solution formée est extraite avec le solvant dans un rapport de 9/1 à 4/6, de préférence de 6/4, et
dans l'étape (f) de l'acétate de plomb, de l'acétate basique de plomb ou du nitrate de plomb ou une solution aqueuse d'hydroxyde de plomb est utilisé.

3. Procédé suivant la revendication 1, **caractérisé en ce que**, à l'étape (a),
les feuilles de Ginkgo biloba sont extraites à une température comprise entre 50 et 65° C environ, avec comme solvant d'extraction soit l'acétone aqueuse avec un ratio en poids acétone/eau compris entre 50/50 et 65/35 et un ratio solides/solvant de 1/6 à 1/20 ; soit avec de l'éthanol aqueux.

4. Procédé suivant la revendication 3, **caractérisé en ce que**, à l'étape (a),
le solvant d'extraction utilisé est l'acétone aqueuse avec un ratio en poids acétone/eau de 60/40 environ.

5. Procédé suivant la revendication 1, **caractérisé en ce que**, à l'étape (b),
le solvant organique de l'extrait est éliminé par évaporation sous pression réduite jusqu'à un taux maximum de solvant restant inférieur à 5 % et jusqu'à obtenir un contenu solide de l'ordre de 15 à 30 %.

6. Procédé suivant la revendication 1, **caractérisé en ce que**, à l'étape (c),
la solution aqueuse concentrée restante est diluée avec de l'eau jusqu'à obtenir un contenu solide de l'ordre de 10 à 20 % en poids et un taux maximum de solvant restant inférieur à 5 %, puis laissée se refroidir sous agitation à une température de l'ordre de 4-12° C pendant au moins une heure.

7. Procédé suivant la revendication 1, **caractérisé en ce que**, à l'étape (d),
la solution aqueuse restante est extraite avec un mélange méthyléthylcétone/acétone avec un ratio en poids de 6/4 environ, en présence de 30 à 40 %, et de préférence 35 %, en poids par rapport au volume de la solution aqueuse, de sulfate d'ammonium, puis, après séparation de phases, on récupère la phase organique

8. Procédé suivant la revendication 1, **caractérisé en ce que**, à l'étape (f),
une solution aqueuse d'acétate basique de plomb (CH₃CO₂)₂Pb.2Pb(OH)₂ à une concentration de l'ordre de 15 à 20 % est ajoutée à la solution ainsi obtenue à l'étape précédente (e), avec un ratio (quantité de Pb/contenu solide) compris entre 0,15 et 0,30.

9. Procédé suivant la revendication 8, **caractérisé en ce que** le ratio (Pb/contenu solide) est compris entre 0,18 et 0,25.

10. Procédé suivant la revendication 1, **caractérisé en ce que**, à l'étape (g), la solution alcoolique aqueuse restante est extraite avec de l'heptane.

11. Procédé selon l'une des revendications précédentes, **caractérisé en ce que** le dit procédé comprend une étape supplémentaire qui consiste à faire passer sur un adsorbant la phase organique obtenue à l'étape d.

12. Procédé selon l'une des revendications 1 à 10, **caractérisé en ce que** le dit procédé comprend une étape supplémentaire qui consiste à faire passer sur un adsorbant la phase organique obtenue à l'étape i.

13. Procédé selon l'une des revendications précédentes, **caractérisé en ce que** le dit procédé comprend une étape supplémentaire qui consiste à faire passer sur un adsorbant la phase organique obtenue à l'étape k.

14. Procédé d'obtention d'un extrait de feuilles de Ginkgo biloba substantiellement dépourvu de polluants organiques persistants, ledit procédé **caractérisé en ce qu'**il comprend les étapes suivantes :
(aa) extraction de feuilles sèches de Ginkgo biloba à une température comprise entre 40 et 100° C environ, avec un solvant d'extraction choisi parmi : l'acétone aqueuse ou un alcool inférieur de 3 atomes de carbone aqueux ;
(ba) élimination du solvant organique de l'extrait jusqu'à un taux maximum de solvant restant inférieur à 10 % ;
(ca) la solution aqueuse concentrée restante est diluée avec de l'eau jusqu'à obtenir un contenu solide de fordre de 5 à 25 % en poids et un taux maximum de solvant restant inférieur à 5 %, puis laissée se refroidir sous agitation à une température inférieure à 25° C pendant au moins une heure et le précipité ainsi formé, consistant en des composés lipophiles, est éliminés ;
(da) la solution aqueuse restante est extraite avec un mélange méthyl éthyl cétone / acétone en présence de sulfate d'ammonium, puis, après séparation de phases, la phase organique est concentrée jusqu'à un contenu solide de 40 à 60 % en poids,
(ea) le concentré obtenu est dilué avec de l'éthanol aqueux et de l'eau de telle sorte que la solution finale contienne environ 50 % en poids d'eau et 50 % en poids d'éthanol et un contenu solide de l'ordre de 10-12 % ;
(fa) une solution aqueuse de sels de plomb est ajoutée à la solution ainsi obtenue à l'étape (e), avec un ratio (quantité de Pb/contenu solide) compris entre 0,15 et 0,3, et le précipité de plomb ainsi formé est retiré ;
(ga) la solution éthanolique aqueuse restante est extraite avec de l'heptane afin de retirer les composés de type alkylphénol et la solution aqueuse alcoolique restante est concentrée sous pression réduite jusqu'à un contenu d'éthanol inférieur à 5 % en poids ;
(ha) la solution obtenue est extraite avec un mélange méthyl éthyl cétone et éthanol, en présence de sulfate d'ammonium ;
(ia) à la phase organique obtenue à l'étape précédente est ajouté du sulfate d'ammonium sous agitation puis, après décantation, on récupère la phase organique qui est ensuite concentrée jusqu'à un contenu solide de l'ordre de 50-70 % en poids ;
(ja) au concentré ainsi obtenu, on ajoute de l'éthanol et de l'eau jusqu'à obtenir un contenu solide de 10 à 12 % , un contenu en éthanol d'environ 75 à 90 %, le reste étant de l'eau, puis on refroidit aux environs de 8-12° C pendant au moins une heure et enfin les insolubles sont éliminés par filtration et on récupère le filtrat ;
(ka) le filtrat ainsi obtenu est concentré sous pression réduite jusqu'à obtenir un contenu solide de 50 à 70 % en poids, et séché dans une étuve sous vide à une température qui ne dépasse pas 60° C ;
et une étape supplémentaire consistant à faire passer une des phases organiques obtenues à l'une des étapes précédentes da, ia ou ja, sur un adsorbant qui est constitué de charbon actif synthétique, et présentant une surface spécifique supérieure ou égale à 1000 m²/g, avec un solvant ou mélange de solvants dont la polarité est comprise entre 5 et 6.

15. Procédé selon la revendication 14, **caractérisé en ce que**, à l'étape (aa)
l'extraction de feuilles sèches de Ginkgo biloba s'effectue à une température comprise entre 50-65° C environ, avec comme solvant d'extraction soit l'acétone aqueuse avec un ratio en poids acétone/eau compris entre 50/50 et 65/35 et un ratio (feuilles sèches/solvant) compris entre 1/6 et 1/20, soit de l'éthanol aqueux.

16. Procédé suivant la revendication 15, **caractérisé en ce que**, à l'étape (aa),
le solvant d'extraction utilisé est l'acétone aqueuse avec un ratio en poids acétone/eau de 60/40 environ.

17. Procédé selon la revendication 14, **caractérisé en ce que**, à l'étape (ba),
l'élimination du solvant organique de l'extrait s'effectue par évaporation sous pression réduite jusqu'à un taux maximum de solvant restant inférieur à 5 % et jusqu'à obtenir un contenu solide de l'ordre de 15 à 30 %.

18. Procédé selon la revendication 14, **caractérisé en ce que**, à l'étape (ca),
la solution aqueuse concentrée restante est diluée avec de l'eau jusqu'à obtenir un contenu solide de l'ordre de 10 à 20 % en poids, puis laissée se refroidir sous agitation à une température de 4-12° C.

19. Procédé selon la revendication 14, **caractérisé en ce que**, à l'étape (da),
la solution aqueuse restante est extraite avec un mélange méthyle éthyle cétone / acétone avec un ratio en poids compris entre 5/5 et 7/3, de préférence de 6/4 environ, en présence de sulfate d'ammonium avec un taux de 30 à 40 %, et de préférence, 35 %, en poids par rapport au volume de la solution aqueuse.

20. Procédé selon la revendication 14, **caractérisé en ce que**, à l'étape (fa),
le sel de plomb est choisi parmi l'acétate de plomb, l'hydroxyde de plomb et l'acétate basique de plomb.

21. Procédé suivant la revendication 14 ou 20, **caractérisé en ce que** le ratio (Pb/contenu solide) est compris entre 0,18 et 0,25.

22. Procédé suivant la revendication 14 ou 20, **caractérisé en ce que**, à l'étape (fa),
une solution aqueuse d'acétate basique de plomb (CH₃CO₂)₂Pb.2Pb(OH)₂ à une concentration de l'ordre de 15 à 20 %, est ajoutée à la solution obtenue à l'étape (ea), avec un ratio (quantité de Pb/contenu solide) compris entre 0,18 et 0,25.

23. Procédé selon la revendication 14, **caractérisé en ce que**, à l'étape (ha),
la solution obtenue est extraite avec un mélange méthyl éthyl cétone et éthanol avec un ratio de l'ordre de 6/4 en poids, en présence de 30 à 40 %, et de préférence 35 % en poids par rapport au volume de la solution aqueuse, de sulfate d'ammonium.

24. Procédé selon la revendication 14, **caractérisé en ce que**, à l'étape (ja), on ajoute de l'éthanol et de l'eau jusqu'à obtenir un contenu en éthanol d'environ 78 à 90 %.

25. Procédé selon l'une des revendications 14 à 24, **caractérisé en ce que** le dit procédé comprend une étape supplémentaire qui consiste à faire passer sur un adsorbant la phase organique obtenue à l'étape da.

26. Procédé selon l'une des revendications 14 à 24, **caractérisé en ce que** le dit procédé comprend une étape supplémentaire qui consiste à faire passer sur un adsorbant la phase organique obtenue à l'étape ia.

27. Procédé selon l'une des revendications 14 à 24, **caractérisé en ce que** le dit procédé comprend une étape supplémentaire qui consiste à faire passer sur un adsorbant la phase organique (le filtrat) obtenue à l'étape ja.

28. Procédé selon l'une des revendications précédentes **caractérisé en ce que** l'adsorbant comprend au moins l'une des caractéristiques ci-dessous :
- il ne comprend pas de groupe fonctionnel ;
- il se présente sous forme sphérique ;
- il présente une surface spécifique supérieure ou égale à 1100 m²/g.

29. Procédé selon l'une des revendications précédentes **caractérisé en ce que** l'adsorbant comprend au moins deux des caractéristiques ci-dessous :
- il ne comprend pas de groupe fonctionnel ;
- il se présente sous forme sphérique ;
- il présente une surface spécifique supérieure ou égale à 1100 m²/g.

30. Procédé selon l'une des revendications précédentes **caractérisé en ce que** l'adsorbant est du charbon actif, sans groupe fonctionnel, sous forme sphérique, avec une surface spécifique minimale supérieure ou égale à 1100 m²/g.

31. Procédé selon l'une des revendications précédentes **caractérisé en ce que** l'adsorbant présente une taille de particules comprise entre 0,2 et 1 mm.

32. Procédé selon l'une des revendications précédentes **caractérisé en ce que** l'adsorbant présente une taille de particules comprise entre 0,3 et 0,9 mm.

33. Procédé selon l'une des revendications précédentes **caractérisé en ce que** l'adsorbant est du charbon actif, sans groupe fonctionnel, sous forme sphérique de diamètre compris entre 0,4 et 0,8 mm, avec une surface spécifique minimale supérieure ou égale à 1200 m²/g avec des diamètres de pore d'environ 8 nm et des volumes de pore d'environ 0,15 cm³/g.

34. Extrait de Ginkgo biloba substantiellement dépourvu de polluants organiques persistants et susceptible d'être obtenu selon un procédé tel que décrit à l'une des revendications précédentes.

35. Extrait de Ginkgo biloba selon la revendication 34 substantiellement dépourvu de polluants organiques persistants et comprenant
- 20 à 30 % en poids de flavones glycosides,
- 2,5 à 4,5 % en poids total de Ginkgolides A, B, C et J (au total),
- 2,0 à 4,0 % en poids de bilobalide,
- moins de 10 ppm d'alkylphénols et moins de 10 % en poids de proanthocyanidines.

36. Extrait de Ginkgo biloba selon la revendication 35 substantiellement dépourvu de polluants organiques persistants et comprenant
- 22 à 26 % en poids de flavones glycosides,
- 2,5 à 4,5 % en poids total de Ginkgolides A, B, C et J,
- 2,0 à 4,0 % en poids de bilobalide,
- moins de 1 ppm d'alkylphénols et moins de 10 % en poids de proanthocyanidines:

37. Extrait de Ginkgo biloba selon la revendication 34 substantiellement dépourvu de polluants organiques persistants et comprenant
- 20 à 30 % en poids de flavones glycosides,
- 2,5 à 4,5 % en poids total de Ginkgolides A, B et C,
- 2,0 à 4,0 % en poids de bilobalide,
- moins de 10 ppm d'alkylphénols et moins de 10 % en poids de proanthocyanidines.

38. Extrait de Ginkgo biloba selon l'une des revendications 34 à 37, avec un taux de chaque PAH inférieur à 5 µg/kg et le TEQ (PCDD/PCDF-PCB) inférieur à 10 pg/g.

39. Extrait de Ginkgo biloba selon l'une des revendications 34 à 38, avec un taux de chaque PAH inférieur à 1 µg/kg et le TEQ (PCDD/PCDF-PCB) inférieur à 1,5 pg/g.

40. Extrait de Ginkgo biloba selon l'une des revendications 34 à 39, avec un taux de chaque PAH inférieur à 0,5 µg/kg et le TEQ (PCDD/PCDF-PCB) inférieur à 1,5 pg/g.

41. Extrait de Ginkgo biloba selon l'une des revendications 34 à 39, avec un taux de PAH inférieur à la limite de quantification (LOQ), et le TEQ (PCDD/PCDF-PCB) inférieur à 1,0 pg/g.

42. Utilisation d'un extrait tel que défini à l'une des revendications 34 à 41, pour la préparation d'un médicament pour le traitement des désordres circulatoires périphériques ou cérébraux, de la démence ou les syndromes de la démence.

43. Composition thérapeutique contenant, à titre de principe actif, un extrait tel que défini à l'une des revendications 34 à 41, en association avec des excipients thérapeutiquement acceptables.

## Patentansprüche

1. Verfahren zur Gewinnung eines Gingko-biloba-Blätter-Extraktes, der im Wesentlichen frei von persistenten organischen Verunreinigungen ist, wobei das Verfahren die folgenden Stufen umfasst:
(a) die Gingko-biloba-Blätter werden bei einer Temperatur von etwa 40 bis 100 °C mit wässrigen Aceton, einem wässrigen Alkohol mit 1 bis 3 Kohlenstoffatomen oder wasserfreiem Methanol extrahiert;
(b) der Hauptteil des organischen Lösungsmittels wird zu einem maximalen Gehalt von 10 Gewichts-% von dem Extrakt abgetrennt, sodass das Wasser im Verlauf der letzten Destillationsstufen zugegeben werden kann;
(c) die zurückbleibende konzentrierte wässrige Lösung wird mit Wasser zu einem Trockensubstanzgehalt von 5 bis 25 Gewichts-% verdünnt, unter kontinuierlichem Rühren auf eine Temperatur von unter 25 °C abkühlen gelassen, ruhen gelassen, bis sich ein Präzipitat bildet, und das resultierende Präzipitat, das aus lipophilen Bestandteilen, die sich nicht gut in Wasser lösen, besteht, wird abgetrennt;
(d) zu der verbleibenden wässrigen Lösung wird Ammoniumsulfat gegeben, und die gebildete Lösung wird mit Methylethylketon oder einem Gemisch aus Methylethylketon und Aceton extrahiert;
(e) der erhaltene Extrakt wird zu einem Trockensubstanzgehalt von 50 bis 70 % konzentriert und das erhaltene Konzentrat wird derart mit Wasser und Ethanol verdünnt, dass eine Lösung erhalten wird, die 50 Gewichts-% Wasser und 50 Gewichts-% Ethanol bei einem Feststoffgehalt von 10 Gewicht-% enthält;
(f) eine wässrige Lösung eines Bleisalzes wird zu der so erhaltenen Lösung gegeben, bis eine Farbänderung von braun nach bernsteinfarben auftritt, und das gebildete Präzipitat wird abgetrennt, oder es wird ein Polyamid anstelle eines Bleisalzes verwendet;
(g) die zurückbleibende wässrig-alkoholische Lösung wird mit einem aliphatischen oder cycloaliphatischen Lösungsmittel mit einem Siedepunkt von 60 bis 100 °C extrahiert, um mehr Alkylphenolverbindungen abzutrennen;
(h) die zurückbleibende wässrig-alkoholische Lösung wird unter vermindertem Druck zu einem maximalen Ethanolgehalt von etwa 5 % konzentriert, und es wird Ammoniumsulfat bis zu einem Gehalt von 20 Gewichts-% zugegeben;
(i) die erhaltene Lösung wird mit einem Gemisch aus Methylethylketon und Ethanol in einem Verhältnis von 8/2 bis 5/5, vorzugsweise von 6/4, extrahiert;
(j) die resultierende organische Phase wird zu einem Feststoffgehalt von 50 bis 70 Gewichts-% konzentriert;
(k) zu dem so erhaltenen Konzentrat gibt man Ethanol, bis ein Ethanolgehalt von wenigstens 80 % erhalten wird, dann kühlt man auf eine maximale Temperatur von 12 °C während wenigstens zwei Stunden, und schließlich werden die unlöslichen Bestandteile durch Filtration entfernt und man gewinnt das Filtrat;
(l) das Filtrat wird unter vermindertem Druck bei einer Temperatur von zwischen 60 und 80 °C bis zum Erhalt eines trockenen Extraktes mit einem Wassergehalt von weniger als 5 % konzentriert und getrocknet,
**dadurch gekennzeichnet, dass** das Verfahren eine zusätzliche Stufe umfasst, die darin besteht, eine der organischen Phasen, die in einer der Stufen d, i oder k erhalten wurden, über ein Adsorptionsmittel, das aus synthetischer Aktivkohle besteht, die eine spezifische Oberfläche von über oder gleich 1000 m²/g aufweist, mit einem Lösungsmittel oder Lösungsmittelgemisch, dessen Polarität zwischen 5 und 6 liegt, zu leiten.

2. Verfahren nach Anspruch 1, **dadurch gekennzeichnet, dass**
in der Stufe (b) das organische Lösungsmittel zu einem maximalen Gehalt von 5 Gewichts-% abgetrennt wird;
in der Stufe (c) die wässrige Lösung mit Wasser zu einem Feststoffgehalt von 15 bis 20 Gewichts-% verdünnt wird und auf eine Temperatur von etwa 10 bis 12 °C abgekühlt wird;
in der Stufe (d) das Ammoniumsulfat zu der Lösung gegeben wird, um einen Gehalt von 30 Gewichts-% zu ergeben, und die gebildete Lösung mit dem Lösungsmittel in einem Verhältnis von 9/1 bis 4/6, vorzugsweise 6/4, extrahiert wird, und
in der Stufe (f) Bleiacetat, basisches Bleiacetat oder Bleinitrat oder eine wässrige Bleihydroxid-Lösung verwendet wird.

3. Verfahren nach Anspruch 1, **dadurch gekennzeichnet, dass** in der Stufe (a)
die Gingko-biloba-Blätter bei einer Temperatur, die etwa zwischen 50 und 65 °C liegt, mit entweder wässrigem Aceton mit einem Gewichtsverhältnis Aceton/Wasser von zwischen 50/50 und 65/35 und einem Verhältnis Feststoffe/Lösungsmittel von 1/6 bis 1/20 oder mit wässrigem Ethanol als Extraktionslösungsmittel extrahiert werden.

4. Verfahren nach Anspruch 3, **dadurch gekennzeichnet, dass** in der Stufe (a) das verwendete Extraktionslösungsmittel wässriges Aceton mit einem Gewichtsverhältnis Aceton/Wasser von etwa 60/40 ist.

5. Verfahren nach Anspruch 1, **dadurch gekennzeichnet, dass** in der Stufe (b)
das organische Lösungsmittel des Extrakts durch Eindampfen unter vermindertem Druck bis zu einem maximalen Gehalt an zurückbleibendem Lösungsmittel von unter 5 % und bis zum Erhalt eines Feststoffgehalts in der Größenordnung von 15 bis 30 % entfernt wird.

6. Verfahren nach Anspruch 1, **dadurch gekennzeichnet, dass** in der Stufe (c)
die zurückbleibende konzentrierte wässrige Lösung mit Wasser verdünnt wird, bis ein Feststoffgehalt in der Größenordnung von 10 bis 20 Gewichts-% und ein maximaler Gehalt an zurückbleibendem Lösungsmittel von unter 5 % erhalten wird, dann unter Rühren bei einer Temperatur in der Größenordnung von 4 bis 12 °C während wenigstens einer Stunde abkühlen gelassen wird.

7. Verfahren nach Anspruch 1, **dadurch gekennzeichnet, dass** in der Stufe (d)
die zurückbleibende wässrige Lösung mit einem Gemisch aus Methylethylketon/ Aceton mit einem Gewichtsverhältnis von etwa 6/4 in Gegenwart von 30 bis 40 % und vorzugsweise 35 %, nach Gewicht, bezogen auf das Volumen der wässrigen Lösung, Ammoniumsulfat extrahiert wird, man danach nach Phasentrennung die organische Phase gewinnt.

8. Verfahren nach Anspruch 1, **dadurch gekennzeichnet, dass** in der Stufe (f)
eine wässrige Lösung von basischem Bleiacetat (CH₃CO₂)₂Pb.2Pb(OH)₂ mit einer Konzentration in der Größenordnung von 15 bis 20 % der Lösung, die so in der vorangehenden Stufe (e) erhalten wurde, mit einem Verhältnis (Menge an Pb/Feststoffgehalt), das zwischen 0,15 und 0,30 liegt, zugesetzt wird.

9. Verfahren nach Anspruch 8, **dadurch gekennzeichnet, dass** das Verhältnis (Pb/Feststoffgehalt) zwischen 0,18 und 0,25 liegt.

10. Verfahren nach Anspruch 1, **dadurch gekennzeichnet, dass** in der Stufe (g) die zurückbleibende wässrig-alkoholische Lösung mit Heptan extrahiert wird.

11. Verfahren nach einem der vorangehenden Ansprüche, **dadurch gekennzeichnet, dass** das Verfahren eine zusätzliche Stufe umfasst, die darin besteht,
die in der Stufe d erhaltene organische Phase über ein Adsorptionsmittel zu leiten.

12. Verfahren nach einem der Ansprüche 1 bis 10, **dadurch gekennzeichnet, dass** das Verfahren eine zusätzliche Stufe umfasst, die darin besteht, die in der Stufe i erhaltene organische Phase über ein Adsorptionsmittel zu leiten.

13. Verfahren nach einem der vorangehenden Ansprüche, **dadurch gekennzeichnet, dass** das Verfahren eine zusätzliche Stufe umfasst, die darin besteht, die in der Stufe k erhaltene organische Phase über ein Adsorptionsmittel zu leiten.

14. Verfahren zur Gewinnung eines Gingko-biloba-Blätter-Extraktes, der im Wesentlichen frei von persistenten organischen Verunreinigungen ist, wobei das Verfahren **dadurch gekennzeichnet ist, dass** es die folgenden Stufen umfasst:
(aa) Extraktion von trockenen Gingko-biloba-Blättern bei einer Temperatur, die zwischen etwa 40 und 100 °C liegt, mit einem Extraktionslösungsmittel, das ausgewählt ist aus: wässrigem Aceton oder einem wässrigen niederen Alkohol mit 3 Kohlenstoffatomen;
(ba) Entfernen des organischen Lösungsmittels aus dem Extrakt bis zu einem maximalen Gehalt an verbleibendem Lösungsmittel von unter 10 %;
(ca) die zurückbleibende konzentrierte wässrige Lösung wird mit Wasser verdünnt, bis ein Feststoffgehalt in der Größenordnung von 5 bis 25 Gewichts-% und ein maximaler Gehalt an zurückbleibendem Lösungsmittel von unter 5 % erhalten wird, danach wird sie unter Rühren bei einer Temperatur von unter 25 °C während wenigstens einer Stunde abkühlen gelassen, und das so gebildete Präzipitat, das aus lipophilen Verbindungen besteht, wird entfernt;
(da) die zurückbleibende wässrige Lösung wird mit einem Gemisch aus Methylethylketon/Aceton in Gegenwart von Ammoniumsulfat extrahiert, dann wird, nach Phasentrennung, die organische Phase bis zu einem Feststoffgehalt von 40 bis 60 Gewichts-% konzentriert;
(ea) das erhaltene Konzentrat wird mit wässrigem Ethanol und Wasser derart verdünnt, dass die Endlösung etwa 50 Gewichts-% Wasser und 50 Gewichts-% Ethanol und einen Feststoffgehalt in der Größenordnung von 10 bis 12 % enthält;
(fa) eine wässrige Lösung von Bleisalzen wird zu der so in der Stufe (e) erhaltenen Lösung bei einem Verhältnis (Menge an Pb/Feststoffgehalt), das zwischen 0,15 und 0,3 liegt, gegeben, und das so gebildete Bleipräzipitat wird entfernt;
(ga) die zurückbleibende wässrig-ethanolische Lösung wird mit Heptan extrahiert, um die Verbindungen des Alkylphenol-Typs zu entfernen, und die verbleibende wässrig-alkoholische Lösung wird unter vermindertem Druck bis zu einem Ethanolgehalt von unter 5 Gewichts-% konzentriert;
(ha) die erhaltene Lösung wird mit einem Gemisch aus Methylethylketon und Ethanol in Gegenwart von Ammoniumsulfat extrahiert;
(ia) zu der in der vorangehenden Stufe erhaltenen organischen Phase wird unter Rühren Ammoniumsulfat gegeben, danach gewinnt man, nach Dekantieren, die organische Phase, die anschließend bis zu einem Feststoffgehalt in der Größenordnung von 50 bis 70 Gewichts-% konzentriert wird;
(ja) zu dem so erhaltenen Konzentrat gibt man Ethanol und Wasser bis zum Erhalt eines Feststoffgehaltes von 10 bis 12 %, eines Gehaltes an Ethanol von etwa 75 bis 90 %, wobei der Rest Wasser ist, dann kühlt man während wenigstens einer Stunde auf etwa 8 bis 12 °C, und schließlich werden die unlöslichen Substanzen durch Filtration entfernt und man gewinnt das Filtrat;
(ka) das so erhaltene Filtrat wird unter vermindertem Druck bis zum Erhalt eines Feststoffgehaltes von 50 bis 70 Gewichts-% konzentriert und in einem Wärmeschrank unter Vakuum bei einer Temperatur, die 60 °C nicht übersteigt, getrocknet;
und eine zusätzliche Stufe, die darin besteht, eine der organischen Phasen, die in einer der vorangehenden Stufen da, ia oder ja erhalten wurden, über ein Adsorptionsmittel, das aus synthetischer Aktivkohle besteht und eine spezifische Ober-fläche von über oder gleich 1000 m²/g aufweist, mit einem Lösungsmittel
oder
Lösungsmittelgemisch, dessen Polarität zwischen 5 und 6 liegt, zu leiten.

15. Verfahren nach Anspruch 14, **dadurch gekennzeichnet, dass** in der Stufe (aa)
die Extraktion von trockenen Gingko-biloba-Blättern bei einer Temperatur, die zwischen etwa 50 und 65 °C liegt, mit entweder wässrigem Aceton mit einem Gewichtsverhältnis Aceton/Wasser von zwischen etwa 50/50 und 65/35 und einem Verhältnis (trockene Blätter/Lösungsmittel) von zwischen 1/6 und 1/20 oder wässrigem Ethanol als Extraktionslösungsmittel erfolgt.

16. Verfahren nach Anspruch 15, **dadurch gekennzeichnet, dass** in der Stufe (aa)
das verwendete Extraktionslösungsmittel wässriges Aceton mit einem Gewichtsverhältnis Aceton/Wasser von etwa 60/40 ist.

17. Verfahren nach Anspruch 14, **dadurch gekennzeichnet, dass** in der Stufe (ba)
das Entfernen des organischen Lösungsmittels des Extraktes durch Einengung unter vermindertem Druck bis zu einem maximalen Gehalt an zurückbleibendem Lösungsmittel von unter 5 % und bis zum Erhalt eines Feststoffgehaltes in der Größenordnung von 15 bis 30 % erfolgt.

18. Verfahren nach Anspruch 14, **dadurch gekennzeichnet, dass** in der Stufe (ca)
die zurückbleibende konzentrierte wässrige Lösung mit Wasser verdünnt wird, bis ein Feststoffgehalt in der Größenordnung von 10 bis 20 Gewichts-% erhalten wird, sie dann unter Rühren auf eine Temperatur von 4 bis 12 °C abkühlen gelassen wird.

19. Verfahren nach Anspruch 14, **dadurch gekennzeichnet, dass** in der Stufe (da)
die zurückbleibende wässrige Lösung mit einem Gemisch aus Methylethylaceton/ Aceton mit einem Gewichtsverhältnis, das zwischen 5/5 und 7/3 liegt, vorzugsweise von etwa 6/4, in Gegenwart von Ammoniumsulfat mit einem Gehalt von 30 bis 40 % und vorzugsweise 35 %, nach Gewicht, bezogen auf das Volumen der wässrigen Lösung, extrahiert wird.

20. Verfahren nach Anspruch 14, **dadurch gekennzeichnet, dass** in der Stufe (fa)
das Bleisalz aus Bleiacetat, Bleihydroxid und basischem Bleiacetat ausgewählt wird.

21. Verfahren nach Anspruch 14 oder 20, **dadurch gekennzeichnet, dass** das Verhältnis (Pb/Feststoffgehalt) zwischen 0,18 und 0,25 liegt.

22. Verfahren nach Anspruch 14 oder 20, **dadurch gekennzeichnet, dass** in der Stufe (fa)
eine wässrige Lösung von basischem Bleiacetat (CH₃CO₂)₂Pb.2Pb(OH)₂ mit einer Konzentration in der Größenordnung von 15 bis 20 % zu der in der Stufe (ea) erhaltenen Lösung mit einem Verhältnis (Menge an Pb/Feststoffgehalt) von zwischen 0,18 und 0,25 gegeben wird.

23. Verfahren nach Anspruch 14, **dadurch gekennzeichnet, dass** in der Stufe (ha)
die erhaltene Lösung mit einem Gemisch aus Methylethylketon und Ethanol mit einem Verhältnis in der Größenordnung von 6/4, nach Gewicht, in Gegenwart von 30 bis 40 % und vorzugsweise 35 %, nach Gewicht, bezogen auf das Volumen der wässrigen Lösung, Ammoniumsulfat extrahiert wird.

24. Verfahren nach Anspruch 14, **dadurch gekennzeichnet, dass** man in der Stufe (ja) Ethanol und Wasser zusetzt, bis ein Gehalt an Ethanol von etwa 78 bis 90 % erreicht wird.

25. Verfahren nach einem der Ansprüche 14 bis 24, **dadurch gekennzeichnet, dass** das Verfahren eine zusätzliche Stufe umfasst, die darin besteht, die in der Stufe da erhaltene organische Phase über ein Adsorptionsmittel zu leiten.

26. Verfahren nach einem der Ansprüche 14 bis 24, **dadurch gekennzeichnet, dass** das Verfahren eine zusätzliche Stufe umfasst, die darin besteht, die in der Stufe ia erhaltene organische Phase über ein Adsorptionsmittel zu leiten.

27. Verfahren nach einem der Ansprüche 14 bis 24, **dadurch gekennzeichnet, dass** das Verfahren eine zusätzliche Stufe umfasst, die darin besteht, die in der Stufe ja erhaltene organische Phase (das Filtrat) über ein Adsorptionsmittel zu leiten.

28. Verfahren nach einem der vorangehenden Ansprüche, **dadurch gekennzeichnet, dass** das Adsorptionsmittel wenigstens eines der unten angeführten Merkmale umfasst:
- es umfasst keine funktionelle Gruppe;
- es liegt in sphärischer Form vor;
- es weist eine spezifische Oberfläche von über oder gleich 1100 m²/g auf.

29. Verfahren nach einem der vorangehenden Ansprüche, **dadurch gekennzeichnet, dass** das Adsorptionsmittel wenigstens zwei der unten angeführten Merkmale umfasst:
- es umfasst keine funktionelle Gruppe;
- es liegt in sphärischer Form vor;
- es weist eine spezifische Oberfläche von über oder gleich 1100 m²/g auf.

30. Verfahren nach einem der vorangehenden Ansprüche, **dadurch gekennzeichnet, dass** das Adsorptionsmittel Aktivkohle, ohne funktionelle Gruppe, in sphärischer Form, mit einer minimalen spezifischen Oberfläche von über oder gleich 1100 m²/g ist.

31. Verfahren nach einem der vorangehenden Ansprüche, **dadurch gekennzeichnet, dass** das Adsorptionsmittel eine Partikelgröße von zwischen 0,2 und 1 mm aufweist.

32. Verfahren nach einem der vorangehenden Ansprüche, **dadurch gekennzeichnet, dass** das Adsorptionsmittel eine Partikelgröße von zwischen 0,3 und 0,9 mm aufweist.

33. Verfahren nach einem der vorangehenden Ansprüche, **dadurch gekennzeichnet, dass** das Adsorptionsmittel Aktivkohle, ohne funktionelle Gruppe, in sphärischer Form mit einem Durchmesser von zwischen 0,4 und 0,8 mm, mit einer minimalen spezifischen Oberfläche von über oder gleich 1200 m²/g, mit Porendurchmessern von etwa 8 nm und Porenvolumina von etwa 0,15 cm³/g ist.

34. Gingko-biloba-Extrakt, der im Wesentlichen frei von persistenten organischen Verunreinigungen ist und nach einem Verfahren, wie es in einem der vor-angehenden Ansprüche beschrieben ist, erhalten werden kann.

35. Gingko-biloba-Extrakt nach Anspruch 34, der im Wesentlichen frei von persistenten organischen Verunreinigungen ist und der umfasst:
- 20 bis 30 Gewichts-% Flavonglycoside,
- 2,5 bis 4,5 Gesamtgewichts-% Gingkolide A, B, C und J (insgesamt),
- 2,0 bis 4,0 Gewichts-% Bilobalid,
- weniger als 10 ppm Alkylphenole und weniger als 10 Gewichts-% Proanthocyanidine.

36. Gingko-biloba-Extrakt nach Anspruch 35, der im Wesentlichen frei von persistenten organischen Verunreinigungen ist und der umfasst:
- 22 bis 26 Gewichts-% Flavonglycoside,
- 2,5 bis 4,5 Gesamtgewichts-% Gingkolide A, B, C und J,
- 2,0 bis 4,0 Gewichts-% Bilobalid,
- weniger als 1 ppm Alkylphenol und weniger als 10 Gewichts-% Proanthocyanidine.

37. Gingko-biloba-Extrakt nach Anspruch 34, der im Wesentlichen frei von persistenten organischen Verunreinigungen ist und der umfasst:
- 20 bis 30 Gewichts-% Flavonglycoside,
- 2,5 bis 4,5 Gesamtgewichts-% Gingkolide A, B und C,
- 2,0 bis 4,0 Gewichts-% Bilobalid,
- weniger als 10 ppm Alkylphenole und weniger als 10 Gewichts-% Proanthocyanidine.

38. Gingko-biloba-Extrakt nach einem der Ansprüche 34 bis 37 mit einem Gehalt von jedem PAH von unter 5 µg/kg und an TEQ (PCDD/PCDF-PCB) von unter 10 pg/g.

39. Gingko-biloba-Extrakt nach einem der Ansprüche 34 bis 38 mit einem Gehalt an PAH von unter 1 µg/kg und an TEQ (PCDD/PCDF-PCB) von unter 1,5 pg/g.

40. Gingko-biloba-Extrakt nach einem der Ansprüche 34 bis 39 mit einem Gehalt an PAH von unter 0,5 µg/kg und an TEQ (PCDD/PCDF-PCB) von unter 1,5 pg/g.

41. Gingko-biloba-Extrakt nach einem der Ansprüche 34 bis 39 mit einem Gehalt an PAH von unterhalb der Bestimmungsgrenze (LOQ) und an TEQ (PCDD/PCDF-PCB) von unter 1,0 pg/g.

42. Verwendung eines Extraktes, wie er in einem der Ansprüche 34 bis 41 definiert ist, zur Herstellung eines Medikaments für die Behandlung von peripheren oder zerebralen Durchblutungsstörungen, von Demenz oder den Syndromen der Demenz.

43. Therapeutische Zusammensetzung, die als Wirkstoff einen Extrakt, wie er in einem der Ansprüche 34 bis 41 definiert ist, in Kombination mit therapeutisch annehmbaren Hilfsstoffen enthält.

## Claims

1. Method for obtaining an extract of *Ginkgo biloba* leaves substantially free of persistent organic pollutants, said method comprising the following stages:
(a) the *Ginkgo biloba* leaves are extracted at a temperature of approximately 40 to 100°C with aqueous acetone, an aqueous alcohol with 1 to 3 carbon atoms or anhydrous methanol;
(b) the majority of the organic solvent is separated from the extract to a maximum content of 10% by weight, in such a way that water can be added during the last distillation stages,
(c) the remaining concentrated aqueous solution is diluted with water to a solid matter content of 5 to 25% by weight, left to cool down, while being stirred, to a temperature below 25°C, left to rest until a precipitate forms and the resulting precipitate, constituted by the lipophilic components that do not dissolve well in water, is separated,
(d) ammonium sulphate is added to the remaining aqueous solution and the solution that has formed is extracted with methyl ethyl ketone or a mixture of methyl ethyl ketone and acetone,
(e) the extract obtained is concentrated to a solid matter content of 50 to 70% and the obtained concentrate is diluted with water and ethanol so as to obtain a solution which contains 50% by weight water and 50% by weight ethanol with a solid matter content of 10% by weight,
(f) an aqueous solution of a lead salt is added to the solution obtained in this way until the colour changes from brown to amber, and the precipitate that has formed is separated, or a polyamide is used instead of a lead salt,
(g) the remaining aqueous alcoholic solution is extracted with an aliphatic or cycloaliphatic solvent with a boiling point of 60 to 100°C in order to further separate the alkylphenol compounds,
(h) the remaining aqueous alcoholic solution is concentrated under reduced pressure to a maximum ethanol content of approximately 5% and ammonium sulphate is added up to a content of 20% by weight,
(i) the solution obtained is extracted with a mixture of methyl ethyl ketone and ethanol in a ratio of 8/2 to 5/5, preferably of 6/4,
(j) the resulting organic phase is concentrated to a solid matter content of 50 to 70% by weight,
(k) ethanol is added to the concentrate obtained in this way until an ethanol content of at least 80% is obtained, then the mixture is cooled down to a maximum temperature of 12°C for at least two hours and finally the insolubles are eliminated by filtration and the filtrate is recovered;
(l) the filtrate is concentrated and dried under reduced pressure at a temperature between 60 to 80°C until a dry extract with a water content of less than 5% is obtained,
**characterized in that** said method comprises an additional stage which consists of passing one of the organic phases obtained in one of stages d, i or k, over an adsorbent which is constituted by synthetic activated carbon having a specific surface area greater than or equal to 1000 m²/g, with a solvent or a mixture of solvents with a polarity comprised between 5 and 6.

2. Method according to claim 1, **characterized in that**
in stage (b) the organic solvent is separated to a maximum content of 5% by weight,
in stage (c) the aqueous solution is diluted with water to a solid matter content of 15 to 20% by weight and cooled down to a temperature of approximately 10 to 12°C,
in stage (d) the ammonium sulphate is added to the solution in order to produce a content of 30% by weight and the solution formed is extracted with the solvent in a ratio of 9/1 to 4/6, preferably of 6/4, and
in stage (f) lead acetate, lead subacetate or lead nitrate or an aqueous solution of lead hydroxide is used.

3. Method according to claim 1, **characterized in that**, in stage (a),
the *Ginkgo biloba* leaves are extracted at a temperature comprised between approximately 50 and 65°C, with either aqueous acetone with an acetone/water weight ratio comprised between 50/50 and 65/35 and a solids/solvent ratio of 1/6 to 1/20; or with aqueous ethanol as extraction solvent.

4. Method according to claim 3, **characterized in that**, in stage (a),
the extraction solvent used is aqueous acetone with an acetone/water weight ratio of approximately 60/40.

5. Method according to claim 1, **characterized in that**, in stage (b),
the organic solvent of the extract is eliminated by evaporation under reduced pressure until a maximum remaining solvent level of less than 5% and until a solid content of the order of 15 to 30% is obtained.

6. Method according to claim 1, **characterized in that**, in stage (c),
the remaining concentrated aqueous solution is diluted with water until a solid content of the order of 10 to 20% by weight and a maximum remaining solvent level of less than 5% is obtained, then allowed to cool down under stirring to a temperature of the order of 4-12°C for at least one hour.

7. Method according to claim 1, **characterized in that**, in stage (d),
the remaining aqueous solution is extracted with a methyl ethyl ketone/acetone mixture with a weight ratio of approximately 6/4, in the presence of 30 to 40%, and preferably 35%, by weight relative to the volume of the aqueous solution, ammonium sulphate, then, after phase separation, the organic phase is recovered.

8. Method according to claim 1, **characterized in that**, in stage (f),
an aqueous solution of lead subacetate (CH₃CO₂)₂Pb.2Pb(OH)₂ at a concentration of the order of 15 to 20% is added to the solution obtained in this way in the previous stage (e), with a (quantity of Pb/solid content) ratio comprised between 0.15 and 0.30.

9. Method according to claim 8, **characterized in that** the (Pb/solid content) ratio is comprised between 0.18 and 0.25.

10. Method according to claim 1, **characterized in that**, in stage (g), the remaining aqueous alcoholic solution is extracted with heptane.

11. Method according to one of the previous claims, **characterized in that** said method comprises an additional stage which consists of passing the organic phase obtained in stage d over an adsorbent.

12. Method according to one of the claims 1 to 10, **characterized in that** said method comprises an additional stage which consists of passing the organic phase obtained in stage i over an adsorbent.

13. Method according to one of the previous claims, **characterized in that** said method comprises an additional stage which consists of passing the organic phase obtained in stage k over an adsorbent.

14. Method for obtaining an extract of *Ginkgo biloba* leaves substantially free of persistent organic pollutants, said method **characterized in that** it comprises the following stages:
(aa) extracting dry *Ginkgo biloba* leaves at a temperature comprised between approximately 40 and 100°C, with an extraction solvent chosen from: aqueous acetone or a lower alcohol with less than 3 carbon atoms;
(ba) eliminating the organic solvent from the extract to a maximum remaining solvent level of less than 10%;
(ca) the remaining concentrated aqueous solution is diluted with water until a solid content of the order of 5 to 25% by weight and a maximum remaining solvent level of less than 5% is obtained, then allowed to cool down under stirring to a temperature less than 25°C for at least one hour and the precipitate formed in this way, consisting of lipophilic compounds, is eliminated;
(da) the remaining aqueous solution is extracted with a methyl ethyl ketone/acetone mixture in the presence of ammonium sulphate, then, after phase separation, the organic phase is concentrated up to a solid content of 40 to 60% by weight,
(ea) the obtained concentrate is diluted with aqueous ethanol and water in such a way that the final solution contains approximately 50% by weight water and 50% by weight ethanol and a solid content of the order of 10-12%;
(fa) an aqueous solution of lead salts is added to the solution obtained in this way in stage (e), with a (quantity of Pb/solid content) ratio comprised between 0.15 and 0.3, and the lead precipitate formed in this way is removed;
(ga) the remaining aqueous ethanol solution is extracted with heptane in order to remove the alkylphenol type compounds and the remaining aqueous alcohol solution is concentrated under reduced pressure to an ethanol content of less than 5% by weight;
(ha) the solution obtained is extracted with a methyl ethyl ketone and ethanol mixture, in the presence of ammonium sulphate;
(ia) ammonium sulphate is added to the organic phase obtained in the previous stage under stirring then, after decantation, the organic phase is recovered, which is then concentrated to a solid content of the order of 50-70% by weight;
(ja) ethanol and water are added to the concentrate obtained in this way until a solid content of 10 to 12% and an ethanol content of approximately 75 to 90% are obtained, the remainder being water, then the mixture is cooled down to approximately 8-12°C for at least one hour and finally the insolubles are eliminated by filtration and the filtrate is recovered;
(ka) the filtrate obtained in this way is concentrated under reduced pressure until a solid content of 50 to 70% by weight is obtained, and dried in an oven under vacuum at a temperature that does not exceed 60°C;
and an additional stage consisting of passing one of the organic phases obtained in one of the previous stages da, ia or ja over an adsorbent which is constituted by synthetic activated carbon having a specific surface area greater than or equal to 1000 m²/g, with a solvent or a mixture of solvents with a polarity comprised between 5 and 6..

15. Method according to claim 14, **characterized in that**, in stage (aa)
the extraction of dry *Ginkgo biloba* leaves is carried out at a temperature comprised between approximately 50-65°C, with either aqueous acetone with an acetone/water weight ratio comprised between 50/50 and 65/35 and a (dry leaves/solvent) ratio comprised between 1/6 and 1/20, or with aqueous ethanol as extraction solvent.

16. Method according to claim 15, **characterized in that**, in stage (aa),
the extraction solvent used is aqueous acetone with an acetone/water weight ratio of approximately 60/40.

17. Method according to claim 14, **characterized in that**, in stage (ba),
the elimination of the organic solvent from the extract is carried out by evaporation under reduced pressure until a maximum remaining solvent level of less than 5% and a solid content of the order of 15 to 30% are obtained.

18. Method according to claim 14, **characterized in that**, in stage (ca),
the remaining concentrated aqueous solution is diluted with water until a solid content of the order of 10 to 20% by weight is obtained, then allowed to cool down under stirring to a temperature of 4-12°C.

19. Method according to claim 14, **characterized in that**, in stage (da),
the remaining aqueous solution is extracted with a methyl ethyl ketone/acetone mixture with a weight ratio comprised between 5/5 and 7/3, preferably approximately 6/4, in the presence of ammonium sulphate at a level of 30 to 40%, and preferably 35% by weight relative to the volume of the aqueous solution.

20. Method according to claim 14, **characterized in that**, in stage (fa),
the lead salt is chosen from lead acetate, lead hydroxide and lead subacetate.

21. Method according to claim 14 or 20, **characterized in that** the (Pb/solid content) ratio is comprised between 0.18 and 0.25.

22. Method according to claim 14 or 20, **characterized in that**, in stage (fa),
an aqueous solution of lead subacetate (CH₃CO₂)₂Pb.2Pb(OH)₂ at a concentration of the order of 15 to 20%, is added to the solution obtained in stage (ea), with a (quantity of Pb/solid content) ratio comprised between 0.18 and 0.25.

23. Method according to claim 14, **characterized in that**, in stage (ha),
the solution obtained is extracted with a methyl ethyl ketone and ethanol mixture with a ratio of the order of 6/4 by weight, in the presence of 30 to 40%, and preferably 35% by weight relative to the volume of the aqueous solution, of ammonium sulphate.

24. Method according to claim 14, **characterized in that**, in stage (ja), ethanol and water are added until an ethanol content of approximately 78 to 90% is obtained.

25. Method according to one of claims 14 to 24, **characterized in that** said method comprises an additional stage which consists of passing the organic phase obtained in stage da over an adsorbent.

26. Method according to one of claims 14 to 24, **characterized in that** said method comprises an additional stage which consists of passing the organic phase obtained in stage ia over an adsorbent.

27. Method according to one of claims 14 to 24, **characterized in that** said method comprises an additional stage which consists of passing the organic phase (the filtrate) obtained in stage ja over an adsorbent.

28. Method according to one of the previous claims **characterized in that** the adsorbent comprises at least one of the characteristics below:
- it does not comprise a functional group;
- it is presented in spherical form;
- it has a specific surface area greater than or equal to 1100 m²/g.

29. Method according to one of the previous claims **characterized in that** the adsorbent comprises at least two of the characteristics below:
- it does not comprise a functional group;
- it is presented in spherical form;
- it has a specific surface area greater than or equal to 1100 m²/g.

30. Method according to one of the previous claims **characterized in that** the adsorbent is activated carbon, without a functional group, in spherical form, with a minimum specific surface area greater than or equal to 1100 m²/g.

31. Method according to one of the previous claims **characterized in that** the adsorbent has a particle size comprised between 0.2 and 1 mm.

32. Method according to one of the previous claims **characterized in that** the adsorbent has a particle size comprised between 0.3 and 0.9 mm.

33. Method according to one of the previous claims **characterized in that** the adsorbent is activated carbon, without a functional group, in spherical form with a diameter comprised between 0.4 and 0.8 mm, with a minimum specific surface area greater than or equal to 1200 m²/g with pore diameters of approximately 8 nm and pore volumes of approximately 0.15 cm³/g.

34. Extract of *Ginkgo biloba* substantially free of persistent organic pollutants and capable of being obtained according to a method as described in one of the previous claims.

35. Extract of *Ginkgo biloba* according to claim 34 substantially free of persistent organic pollutants and comprising
- 20 to 30% by weight flavone glycosides,
- 2.5 to 4.5% by total weight ginkgolides A, B, C and J (in total),
- 2.0 to 4.0% by weight bilobalide,
- less than 10 ppm of alkylphenols and less than 10% by weight anthocyanidine precursors.

36. Extract of *Ginkgo biloba* according to claim 35 substantially free of persistent organic pollutants and comprising
- 22 to 26% by weight flavone glycosides,
- 2.5 to 4.5% by total weight ginkgolides A, B, C and J,
- 2.0 to 4.0% by weight bilobalide,
- less than 1 ppm of alkylphenols and less than 10% by weight anthocyanidine precursors.

37. Extract of *Ginkgo biloba* according to claim 34 substantially free of persistent organic pollutants and comprising
- 20 to 30% by weight flavone glycosides,
- 2.5 to 4.5% by total weight ginkgolides A, B and C,
- 2.0 to 4.0% by weight bilobalide,
- less than 10 ppm of alkylphenols and less than 10% by weight anthocyanidine precursors.

38. Extract of *Ginkgo biloba* according to one of claims 34 to 37, with a level of each PAH less than 5 µg/kg and the TEQ (PCDD/PCDF-PCB) less than 10 pg/g.

39. Extract of *Ginkgo biloba* according to one of claims 34 to 38, with a level of each PAH less than 1 µg/kg and the TEQ (PCDD/PCDF-PCB) less than 1.5 pg/g.

40. Extract of *Ginkgo biloba* according to one of claims 34 to 39, with a level of each PAH less than 0.5 µg/kg and the TEQ (PCDD/PCDF-PCB) less than 1.5 pg/g.

41. Extract of *Ginkgo biloba* according to one of claims 34 to 40, with a level of PAH less than the limit of quantification (LOQ), and the TEQ (PCDD/PCDF-PCB) less than 1.0 pg/g.

42. Use of an extract as defined in one of claims 34 to 41, for the preparation of a medicament for the treatment of peripheral or cerebral circulatory disorders, dementia or dementia syndromes.

43. Therapeutic composition containing, as active ingredient, an extract as defined in one of claims 34 to 41, in combination with therapeutically acceptable excipients.
